# EUROPEAN PATENT SPECIFICATION

(11) **EP 1 765 775 B1**
(45) Date of publication and mention of the grant of the patent: **29.04.2009**
(21) Application number: 05755243.2
(22) Date of filing: 17.06.2005
(51) Int. Cl.: C07D 209/42, C07D 401/04, C07D 401/14, A61K 31/404, A61P 29/00

(54) **INDOLES USEFUL IN THE TREATMENT OF INFLAMMATION**
ZUR BEHANDLUNG VON ENTZÜNDUNGEN GEEIGNETE INDOLE
INDOLES UTILES DANS LE TRAITEMENT DE L'INFLAMMATION

(30) Priority: 18.06.2004 US 580403 P
(43) Date of publication of application: 28.03.2007
(73) Proprietor: Biolipox AB, 171 65 Solna (SE)
(72) Inventor: OLOFSSON, Kristofer, c/o Biolipox AB, Solna SE-171 65 (SE); SUNA, E, c/o Latvian Inst. of Organic Synthesis, LV-1006 Riga (LV); PELCMAN, Benjamin; Biolipox AB, Solna SE-171 65 (SE); OZOLA, Vita; Latvian Inst. of Organic Synthesis, LV-1006 Riga (LV); KATKEVICS, M.; Latvian Inst. of Organic Synthesis, LV-1006 Riga (LV); KALVINS, Ivars; Latvian Inst. of Organic Synthesis, LV-1006 Riga (LV); SCHAAL, Wesley; Biolipox AB, S-171 65 Solna (SE)
(74) Representative: McNeeney, Stephen Phillip
(86) International application number: PCT/GB2005/002404
(87) International publication number: WO 2005/123675

(56) References cited:
- WO-A-00/46195

## Description

### Field of the Invention

This invention relates to novel pharmaceutically-useful compounds, which compounds are useful as inhibitors of enzymes belonging to the membrane-associated proteins in the eicosanoid and glutathione metabolism (MAPEG) family. Members of the MAPEG family include the microsomal prostaglandin E synthase-1 (mPGES-1), 5-lipoxygenase-activating protein (FLAP), leukotriene C₄ synthase and microsomal glutathione S-transferases (MGST1, MGST2 and MGST3). The compounds are of potential utility in the treatment of inflammatory diseases including respiratory diseases. The invention also relates to the use of such compounds as medicaments, to pharmaceutical compositions containing them, and to synthetic routes for their production

### Background of the Invention

There are many diseases/disorders that are inflammatory in their nature. One of the major problems associated with existing treatments of inflammatory conditions is a lack of efficacy and/or the prevalence of side effects (real or perceived).

Inflammatory diseases that affect the population include asthma, inflammatory bowel disease, rheumatoid arthritis, osteoarthritis, rhinitis, conjunctivitis and dermatitis.

Inflammation is also a common cause of pain. Inflammatory pain may arise for numerous reasons, such as infection, surgery or other trauma. Moreover, several diseases including malignancies and cardioavascular diseases are known to have inflammatory components adding to the symptomatology of the patients.

Asthma is a disease of the airways that contains elements of both inflammation and bronchoconstriction. Treatment regimens for asthma are based on the severity of the condition. Mild cases are either untreated or are only treated with inhaled β-agonists which affect the bronchoconstriction element, whereas patients with more severe asthma typically are treated regularly with inhaled corticosteroids which to a large extent are antiinflammatory in their nature.

Another common disease of the airways with inflammatory and bronchoconstrictive components is chronic obstructive pulmonary disease (COPD). The disease is potentially lethal, and the morbidity and mortality from the condition is considerable. At present, there is no known pharmacological treatment capable of changing the course of the disease.

The cyclooxygenase (COX) enzyme exists in two forms, one that is constitutively expressed in many cells and tissues (COX-1), and one that is induced by pro-inflammatory stimuli, such as cytokines, during an inflammatory response (COX-2). COXs metabolise arachidonic acid to the unstable intermediate prostaglandin H₂ (PGH₂). PGH₂ is further metabolised to other prostaglandins including PGE₂, PGF_{2α}, PGD₂, prostacyclin and thromboxane A₂. These arachidonic acid metabolites are known to have pronounced physiological and pathophysiological activity including pro-inflammatory effects.

PGE₂ in particular is known to be a strong pro-inflammatory mediator, and is also known to induce fever and pain. Consequently, numerous drugs have been developed with a view to inhibiting the formation of PGE₂, including "NSAIDs" (non-steroidal antiinflammatory drugs) and "coxibs" (selective COX-2 inhibitors). These drugs act predominantly by inhibition of COX-1 and/or COX-2, thereby reducing the formation of PGE₂.

However, the inhibition of COXs has the disadvantage that it results in the reduction of the formation of all metabolites of arachidonic acid, some of which are known to have beneficial properties. In view of this, drugs which act by inhibition of COXs are therefore known/suspected to cause adverse biological effects. For example, the non-selective inhibition of COXs by NSAIDs may give rise to gastrointestinal side-effects and affect platelet and renal function. Even the selective inhibition of COX-2 by coxibs, whilst reducing such gastrointestinal side-effects, is believed to give rise to cardiovascular problems.

An alternative treatment of inflammatory diseases that does not give rise to the above-mentioned side effects would thus be of real benefit in the clinic. In particular, a drug that inhibits (preferably selectively) the transformation of PGH₂ to the pro-inflammatory mediator PGE₂ might be expected to reduce the inflammatory response in the absence of a corresponding reduction of the formation of other, beneficial arachidonic acid metabolites. Such inhibition would accordingly be expected to alleviate the undesirable side-effects mentioned above.

PGH₂ may be transformed to PGE₂ by prostaglandin E synthases (PGES). Two microsomal prostaglandin E synthases (mPGES-1 and mPGES-2), and one cytosolic prostaglandin E synthase (cPGES) have been described.

The leukotrienes (LTs) are formed from arachidonic acid by a set of enzymes distinct from those in the COX / PGES pathway. Leukotriene B4 is known to be a strong proinflammatory mediator, while the cysteinyl-containing leukotrienes C₄, D₄ and E₄ (CysLTs) are mainly very potent bronchoconstrictors and have thus been implicated in the pathobiology of asthma. The biological activities of the CysLTs are mediated through two receptors designated CysLT₁ and CysLT₂. As an alternative to steroids, leukotriene receptor antagonists (LTRas) have been developed in the treatment of asthma. These drugs may be given orally, but do not control inflammation satisfactorily. The presently used LTRas are highly selective for CysLT₁. It may be hypothesised that better control of asthma, and possibly also COPD, may be attained if the activity of both of the CysLT receptors could be reduced. This may be achieved by developing unselective LTRas, but also by inhibiting the activity of proteins, e.g. enzymes, involved in the synthesis of the CysLTs. Among these proteins, 5-lipoxygenase, 5-lipoxygenase-activating protein (FLAP), and leukotriene C₄ synthase may be mentioned. A FLAP inhibitor would also decrease the formation of the proinflammatory LTB₄.

mPGES-1, FLAP and leukotriene C₄ synthase belong to the membrane-associated proteins in the eicosanoid and glutathione metabolism (MAPEG) family. Other members of this family include the microsomal glutathione S-transferases (MGST1, MGST2 and MGST3). For a review, c.f. P.-J. Jacobsson et al in Am. J. Respir. Crit. Care Med. 161, S20 (2000). It is well known that compounds prepared as antagonists to one of the MAPEGs may also exhibit inhibitory activity towards other family members, *c*.*f*. J. H Hutchinson et al in J. Med. Chem. 38, 4538 (1995) and D. Claveau et al in J. Immunol. 170, 4738 (2003). The former paper also describes that such compounds may also display notable cross-reactivity with proteins in the arachidonic acid cascade that do not belong to the MAPEG family, e.g. 5-lipoxygenase.

Thus, agents that are capable of inhibiting the action of mPGES-1, and thus reducing the formation of the specific arachidonic acid metabolite PGE₂, are likely to be of benefit in the treatment of inflammation. Further, agents that are capable of inhibiting the action of the proteins involved in the synthesis of the leukotrienes are also likely to be of benefit in the treatment of asthma and COPD.

### Prior Art

Various indole-2-carboxylates, and derivatives thereof, have been disclosed in international patent applications WO 01/30343, WO 96/03377, WO 01/00197 and WO 99/33800, US patents Nos. 5,189,054 and 4,960,786, European patent application EP 483 881 and Italian Patent No. 1303260. However, none of these documents disclose or suggest the use of the indole-2-carboxylates in the treatment of inflammation.

Similar indole-2-carboxylates have been disclosed for potential use in the treatment of inflammation in international patent applications WO 99/07678, WO 99/07351, WO 00/46198, WO 00/46197, WO 00/46195, WO 00/46199, WO 96/18393, WO 02/30895, WO 99/05104, WO 01/32621 and WO 2005/005415, US patents Nos. 5,081,145 and 5,081,138 and European patent applications EP 166 591 and EP 985 666. However, none of these documents disclose such compounds in which an aromatic group is directly attached to the ring system via the indole nitrogen.

International patent application WO 94/13662 and European patent application EP 186 367 also mention indoles for potential use in the treatment of inflammation. However, these documents do not mention or suggest compounds in which the benzenoid moiety of the indole is substituted with an aromatic ring.

International patent applications WO 94/14434, WO 99/43672, WO 98/08818, WO 99/43654 and WO 99/43651, and US patents Nos. 6,500,853 and 6,630,496 also describe structurally similar indoles for such potential use. However, there is no specific disclosure in any of these documents of indole-2-carboxylates in which an aromatic group is directly attached via the indole nitrogen.

### Disclosure of the Invention

According to the invention there is provided a compound of formula I, 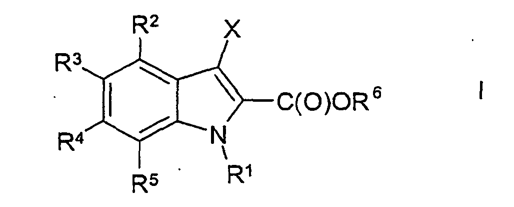 wherein
X and R¹ independently represent an aryl group or a heteroaryl group, both of which groups are optionally substituted by one or more substituents selected from A;
one of the groups R², R³, R⁴ and R⁵ represents an aryl group or a heteroaryl group (both of which are optionally substituted by one or more substituents selected from A) and:
a) the other groups are independently selected from hydrogen, G¹, an aryl group, a heteroaryl group (which latter two groups are optionally substituted by one or more substituents selected from A), C₁₋₈ alkyl and a heterocycloalkyl group (which latter two groups are optionally substituted by one or more substituents selected from G¹ and/or Z¹); and/or
b) any two other groups which are adjacent to each other are optionally linked to form, along with two atoms of the essential benzene ring in the compound of formula I, a 3- to 8-membered ring, optionally containing 1 to 3 heteroatoms and/or 1 to 3 double bonds, which ring is itself optionally substituted by one or more substituents selected from halo, -R⁶, -OR⁶ and =O;

A represents, on each occasion when mentioned above:
I) an aryl group or a heteroaryl group, both of which are optionally substituted by one or more substituents selected from B;
II) C₁₋₈ alkyl or a heterocycloalkyl group, both of which are optionally substituted by one or more substituents selected from G¹ and/or Z¹;
III) a G¹ group; or
IV) two A substituents may be linked together to form, along with at least two (e.g. adjacent) atoms of the aryl or heteroaryl group to which the two A substituents are attached, a further 3- to 5-membered ring, which ring optionally contains 1 to 3 (e.g. 1 or 2) hetereoatoms and/or 1 to 2 (e.g. 1) double bonds, and which is optionally substituted by halo or C₁₋₈ alkyl, which latter group is optionally substituted by halo;

R⁶ represents, on each occasion when mentioned above:
I) hydrogen;
II) an aryl group or a heteroaryl group, both of which are optionally substituted by one or more substituents selected from B; or
III) C₁₋₈ alkyl or a heterocycloalkyl group, both of which are optionally substituted by one or more substituents selected from G¹ and/or Z¹;
G¹ represents, on each occasion when mentioned above, halo, cyano, -N₃, -NO₂, -ONO₂ or -A¹-R⁷;
wherein A¹ represents a single bond or a spacer group selected from -C(O)A²-, -S(O)ₙA³-, -N(R⁸)A⁴- or -OA⁵-, in which:
A² and A³ independently represent a single bond, -O-, -N(R⁸)- or -C(O)-;
A⁴ and A⁵ independently represent a single bond, -C(O)-, -C(O)N(R⁸)-, -C(O)O- -S(O)ₙ- or -S(O)ₙN(R⁸)-;
Z¹ represents, on each occasion when mentioned above, =O, =S, =NOR⁷, =NS(O)ₙN(R⁸)(R⁷), =NCN or =C(H)NO₂;

B represents, on each occasion when mentioned above:
I) an aryl group or a heteroaryl group, both of which are optionally substituted by one or more substituents selected from G², methylenedioxy, difluoromethylenedioxy and/or dimethylmethylenedioxy;
II) C₁₋₈ alkyl or a heterocycloalkyl group, both of which are optionally substituted by one or more substituents selected from G² and/or Z²;
III) a G² group; or
IV) methylenedioxy, difluoromethylenedioxy or dimethylmethylenedioxy;
   G² represents, on each occasion when mentioned above, halo, cyano, -N₃, -NO₂, -ONO₂ or -A⁶-R⁹;
   wherein A⁶ represents a single bond or a spacer group selected from -C(O)A⁷-, -S(O)ₙA⁸-, -N(R¹⁰)A⁹- or -OA¹⁰-, in which:
   A⁷ and A⁸ independently represent a single bond, -O-, -N(R¹⁰)- or -C(O)-;
   A⁹ and A¹⁰ independently represent a single bond, -C(O)-, -C(O)N(R¹⁰)-, -C(O)O-, -S(O)ₙ- or -S(O)ₙN(R¹⁰)-;
   Z² represents, on each occasion when mentioned above, =O, =S, NOR⁹, =NS(O)ₙN(R¹⁰)(R⁹), =NCN or =C(H)NO₂;
   R⁷, R⁸, R⁹ and R¹⁰ are independently selected from:
   i) hydrogen;
   ii) an aryl group or a heteroaryl group, both of which are optionally substituted by one or more substituents selected from G³, methylenedioxy, difluoromethylenedioxy and/or dimethylmethylenedioxy;
   iii) C₁₋₈ alkyl or a heterocycloalkyl group, both of which are optionally substituted by G³ and/or Z³; or
      any pair of R⁷ and R⁸, or R⁹ and R¹⁰, may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from G³ and/or Z³;
      G³ represents, on each occasion when mentioned above, halo, cyano, -N₃,
   -NO₂, -ONO₂ or -A¹¹-R¹¹;
   wherein A¹¹ represents a single bond or a spacer group selected from -C(O)A¹²-, -S(O)ₙA¹³-, -N(R¹²)A¹⁴- or -OA¹⁵-, in which:
   A¹² and A¹³ independently represent a single bond, -O-, N(R¹²)- or -C(O)-;
   A¹⁴ and A¹⁵ independently represent a single bond, -C(O)-, -C(O)N(R¹²)-, -C(O)O-, -S(O)ₙ- or -S(O)ₙN(R¹²)-;
   Z³ represents, on each occasion when mentioned above, =O, =S, =NOR¹¹, =NS(O)ₙN(R¹²)(R¹¹), =NCN or =C(H)NO₂;
   n represents, on each occasion when mentioned above, 1 or 2;
   R¹¹ and R¹² are independently selected from:
   i) hydrogen;
   ii) C₁₋₆ alkyl or a heterocycloalkyl group, both of which groups are optionally substituted by one or more substituents selected from halo, C₁₋₄ alkyl, -N(R¹³)(R¹⁴), -O(R¹³) and =O; and
   iii) an aryl or heteroaryl group, both of which are optionally substituted by one or more substituents selected from halo, C₁₋₄ alkyl, -N(R¹³)(R¹⁴) and -OC(R¹³); or
      any pair R¹¹ and R¹² may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from halo, C₁₋₄ alkyl, -N(R¹³)(R¹⁴), -O(R¹³) and =O;
      R¹³ and R¹⁴ are independently selected from hydrogen and C₁₋₄ alkyl, which latter group is optionally substituted by one or more halo groups;
      or a pharmaceutically-acceptable salt thereof,
      which compounds and salts are referred to hereinafter as "the compounds of the invention".

Pharmaceutically-acceptable salts include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo*, by freeze-drying or by filtration). Salts may also be prepared by exchanging a counter-ion of a compound of the invention in the form of a salt with another counter-ion, for example using a suitable ion exchange resin.

Compounds of the invention may contain double bonds and may thus exist as *E* (*entgegen*) and *Z* (*zusammen*) geometric isomers about each individual double bond. All such isomers and mixtures thereof are included within the scope of the invention.

Compounds of the invention may also exhibit tautomerism. All tautomeric forms and mixtures thereof are included within the scope of the invention.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

As used herein C_{1-q} alkyl groups (where q is the upper limit of the range) defined herein may be straight-chain or, when there is a sufficient number (i.e. a minimum of two or three, as appropriate) of carbon atoms, be branched-chain, and/or cyclic (so forming a C_{3-q} cycloalkyl group). C_{3-q} cycloalkyl groups that may be mentioned include monocyclic or bicyclic alkyl groups, which cycloalkyl groups may further be bridged. Further, when there is a sufficient number (i.e. a minimum of four) of carbon atoms, such groups may also be part cyclic. Such alkyl groups may also be saturated or, when there is a sufficient number (i.e. a minimum of two) of carbon atoms, be unsaturated (forming, for example, a C_{3-q} cycloalkenyl, a C₈ cycloalkynyl or, more particularly, a C_{2-q} alkenyl or a C_{2-q} alkynyl group). Further, in the case where the substituent is another cyclic compound, then the cyclic substituent may be attached through a single atom on the cycloalkyl group, forming a so-called "spiro"-compound.

The term "halo", when used herein, includes fluoro, chloro, bromo and iodo.

Heterocycloalkyl groups that may be mentioned include those in which at least one (e.g. one to four) of the atoms in the ring system is other than carbon (i.e. a heteroatom), and in which the total number of atoms in the ring system is between three and twelve (e.g. between five and ten). Further, such heterocycloalkyl groups may be saturated or unsaturated containing one or more double and/or triple bonds, forming for example a C_{2-q} (e.g. C_{3-q}) heterocycloalkenyl (where q is the upper limit of the range) or a C_{3-q} heterocycloalkynyl group. C_{2-q} heterocycloalkyl groups that may be mentioned include aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, oxetanyl, oxiranyl, piperazinyl, piperidinyl, pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl, thietanyl, thiiranyl, thiolanyl, thiomorpholinyl, trithianyl (including 1,3,5-trithianyl), tropanyl and the like. Other heterocycloalkyl groups that may be mentioned include 7-azabicyclo[2.2.1]heptanyl, 6-azabicyclo[3.1.1]heptanyl, 6-azabicyclo-[3.2.1]octanyl, 8-azabicyclo[3.2.1]-octanyl, 7-oxabicyclo[2.2.1]heptanyl and 6-oxabicyclo[3.2.1]octanyl. Heterocycloalkyl groups that may be mentioned include monocyclic and bicyclic heterocycloalkyl groups, which groups may further be bridged. Substituents on heterocycloalkyl groups may, where appropriate, be located on any atom in the ring system including a heteroatom. Further, in the case where the other substituent is another cyclic compound, then the cyclic compound may be attached through a single atom on the heterocycloalkyl group, forming a so-called "spiro"-compound. The point of attachment of heterocycloalkyl groups may be via any atom in the ring system including (where appropriate) a heteroatom (such as a nitrogen atom), or an atom on any fused carbocyclic ring that may be present as part of the ring system. Heterocycloalkyl groups may also be in the *N-* or *S*- oxidised form.

For the avoidance of doubt, the term "bicyclic", when employed in the context of cycloalkyl and heterocycloalkyl groups refers to such groups in which the second ring is formed between two adjacent atoms of the first ring. The term "bridged", when employed in the context of cycloalkyl or heterocycloalkyl groups refers to monocyclic or bicyclic groups in which two non-adjacent atoms are linked by either an alkylene or heteroalkylene chain (as appropriate).

Aryl groups that may be mentioned include C₆₋₁₃ (e.g. C₆₋₁₀) aryl groups. Such groups may be monocyclic or bicyclic and have between 6 and 13 (e.g. 10) ring carbon atoms, in which at least one ring is aromatic. C₆₋₁₃ aryl groups include phenyl, naphthyl and the like, such as fluorenyl and, more particularly, 1,2,3,4-tetrahydronaphthyl, indanyl, and indenyl. The point of attachment of aryl groups may be *via* any atom of the ring system. However, when aryl groups are bicyclic or tricyclic, they are preferably linked to the rest of the molecule *via* an aromatic ring.

Heteroaryl groups that may be mentioned include those which have between 5 and 10 members. Such groups may be monocyclic, bicyclic or tricyclic, provided that at least one of the rings is aromatic and wherein at least one (e.g. one to four) of the atoms in the ring system is other than carbon (i.e. a heteroatom). Heterocyclic groups that may be mentioned include acridinyl, benzimidazolyl, benzodioxanyl, benzodioxepinyl, benzodioxolyl (including 1,3-benzodioxolyl), benzofuranyl, benzofurazanyl, benzothiazolyl (including 2,1,3-benzothiazolyl), benzoxadiazolyl (including 2,1,3-benzoxadiazolyl), benzoxazinyl (including 3,4-dihydro-2*H*-1,4-benzoxazinyl), benzoxazolyl, benzimidazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-a]pyridyl, indazolyl, indolinyl, indolyl, isobenzofuranyl, isochromanyl, isoindolinyl, isoindolyl, isoquinolinyl, isothiaziolyl, isoxazolyl, naphthyridinyl (including 1,5-naphthyridinyl and 1,8-naphthyridinyl), oxadiazolyl (including 1,2,3-oxadiazolyl, 1,2,4-oxadiazolyl and 1,3,4-oxadiazolyl), oxazolyl, phenazinyl, phenothiazinyl, phthalazinyl, pteridinyl, purinyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3,4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1,2,4-thiadiazolyl and 1,3,4-thiadiazolyl), thiazolyl, thiochromanyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be via any atom in the ring system including (where appropriate) a heteroatom (such as a nitrogen atom), or an atom on any fused carbocyclic ring that may be present as part of the ring system. However, when heteroaryl groups are bicyclic or tricyclic, they are preferably linked to the rest of the molecule via an aromatic ring. Heteroaryl groups may also be in the *N-* or *S*- oxidised form.

Heteroatoms that may be mentioned include phosphorus, silicon, boron, tellurium, preferably, selenium and, more preferably oxygen, nitrogen and/or sulfur.

For the avoidance of doubt, optionally substituted methylenedioxy groups, when attached to a ring system, are formed between any two adj acent atoms of the ring system.

For the avoidance of doubt, in cases in which the identity of two or more substituents in a compound of the invention may be the same, the actual identities of the respective substituents are not in any way interdependent. For example, in the situation in which R¹ and X are both aryl groups substituted by one or more C₁₋₈ alkyl groups, the alkyl groups in question may be the same or different. Similarly, when groups are substituted by more than one substituent as defined herein, the identities of those individual substituents are not to be regarded as being interdependent. For example, when X and/or R¹ represents e.g. an aryl group substituted by G¹ in addition to, for example, C₁₋₈ alkyl, which latter group is substituted by G¹, the identities of the two G¹ groups are not to be regarded as being interdependent.

Compounds of the invention that may be mentioned include those in which:
A² and A³ independently represent a single bond, -O- or -N(R⁸)-;
Z¹ represents, on each occasion when mentioned above, =O, =NOR⁷, =NS(O)ₙN(R⁸)(R⁷), =NCN or =C(H)NO₂;
A⁷ and A⁸ independently represent a single bond, -O- or N(R¹⁰)-;
Z² represents, on each occasion when mentioned above, =O, =NOR⁹, =NS(O)ₙN(R¹⁰)(R⁹), =NCN or =C(H)NO₂;
A¹² and A¹³ independently represent a single bond, -O- or -N(R¹²)-; and/or
Z³ represents, on each occasion when mentioned above, =O, =NOR¹¹, =NS(O)ₙN(R¹²)(R¹¹), =NCN or =C(H)NO₂.

Preferred compounds of the invention include those in which:
G¹ represents halo, cyano, -N₃, -NO₂ or -A¹-R⁷;
A⁴ and A⁵ independently represent a single bond, -C(O)-, -C(O)N(R⁸)- or -C(O)O-;
Z¹ represents =NOR⁷, =NCN or, preferably, =O;
G² represents cyano, -N₃ or, more preferably, halo, -NO₂ or -A⁶-R⁹;
A⁶ represents -N(R¹⁰)A⁹- or -OA¹⁰-;
A⁹ represents -C(O)N(R¹⁰)-, -C(O)O- or, more preferably, a single bond or -C(O)-;
A¹⁰ represents A⁹ and, preferably, a single bond;
Z² represents =NOR⁹ or =NCN or, more preferably, =O;
G³ represents halo, -NO₂ or -A¹¹-R¹¹;
A¹¹ represents -N(R¹²)- or -O-;
Z³ represents =O;
n represents 2;
   when either of R¹¹ and R¹² represent optionally substituted C₁₋₆ alkyl, the optional substituent is one or more halo groups;
   when either of R¹³ and R¹⁴ represent optionally substituted C₁₋₄ alkyl, the optional substituent is one or more fluoro groups.

Preferred compounds of the invention include those in which R¹, X and (when they represent an aryl or a heteroaryl group) R², R³, R⁴ and/or R⁵ represent an optionally substituted phenyl, naphthyl, pyrrolyl, furanyl, thienyl, pyrazolyl, imidazolyl (e.g 1-imidazolyl, 2-imidazolyl or 4-imidazolyl), oxazolyl, isoxazolyl, thiazolyl, pyridyl (e.g. 2-pyridyl, 3-pyridyl or 4-pyridyl), indazolyl, indolyl, indolinyl, isoindolinyl, quinolinyl, 1,2,3,4-tetrahydroquinolinyl, isoquinolinyl, 1,2,3,4-tetrahydroisoquinolinyl, quinolizinyl, benzofuranyl, isobenzofuranyl, chromanyl, benzothienyl, pyridazinyl, pyrimidinyl, pyrazinyl, indazolyl, benzimidazolyl, quinazolinyl, quinoxalinyl, 1,3-benzodioxolyl, benzothiazolyl, and/or benzodioxanyl, group. Other groups that may be mentioned include optionally substituted 5,6,7,8-tetrahydroquinolinyl, 5,6,7,8-tetrahydroisoquinolinyl and tetrazolyl.

Preferred values of R¹ include optionally substituted phenyl, naphthyl and pyridyl.

Preferred values of X include optionally substituted phenyl and pyridyl.

Preferred values of R², R³, R⁴ and R⁵, when any one represents an aryl or a heteroaryl group, include optionally substituted phenyl and pyridyl.

Optional substituents on such R¹, X, R², R³, R⁴ and R⁵ groups are preferably selected from:
cyano;
heterocycloalkyl, such as a 4 to 8 (e.g. 5 or 6) membered nitrogen-containing heterocycloalkyl group optionally containing a further heteroatom (e.g. a nitrogen or oxygen heteroatom) and optionally substituted by one or more halo or C₁₋₃ alkyl (e.g. methyl) group, so forming, for example, a pyrrolidinyl, piperidinyl, piperazinyl or morpholinyl group; and, more preferably selected from:
   halo (e.g. fluoro, chloro or bromo);
   -NO₂;
   C₁₋₆ alkyl, which alkyl group may be linear or branched (e.g. C₁₋₄ alkyl (including methyl, ethyl, *n*-propyl, isopropyl, *n*-butyl or *t*-butyl), *n*-pentyl, isopentyl, *n*-hexyl or isohexyl), cyclic (e.g. cyclopropyl, cyclobutyl, cyclopentyl or cyclohexyl), part-cyclic (e.g. cyclopropylmethyl), unsaturated (e.g. 1-propenyl, 2-propenyl, 1-butenyl, 2-butenyl, 3-butenyl, 1-pentenyl, 2-pentenyl, 4-pentenyl or 5-hexenyl) and/or optionally substituted with one or more halo (e.g. fluoro) group (so forming, for example, fluoromethyl, difluoromethyl or trifluoromethyl);
   -OR¹⁵; and
   -N(R¹⁵)R¹⁶;
wherein R¹⁵ and R¹⁶ independently represent, on each occasion when mentioned above, H or C₁₋₆ alkyl, such as methyl, ethyl, *n*-propyl, isopropyl, *n*-butyl or *t*-butyl (which alkyl groups are optionally substituted by one or more halo (e.g. fluoro) groups (so forming, for example, trifluoromethyl)).

Preferred values of R⁶ include C₁₋₄ alkyl and, particularly, H.

More preferred compounds include those in which:
X represents an aryl group such as a phenyl group or a heteroaryl group such as a pyridyl group, both of which are optionally substituted by one or two A groups;
R¹ represents an aryl group such as a phenyl or naphthyl group or a heteroaryl group such as a pyridyl group, both of which are optionally substituted by one or two A groups;
R³ and R⁴ independently represent G¹ or, more preferably, hydrogen, an aryl group, such as a phenyl group, or a heteroaryl group such as a pyridyl group, which latter two groups are optionally substituted by one or two A groups;
at least one of R³ and R⁴ represents optionally substituted aryl or heteroaryl, and up to one other represents G¹ or, more preferably, hydrogen;
when R³ or R⁴ represents an aryl or heteroaryl group, then the other substituents on the essential benzene ring of the compound of formula I (i.e. R², R⁵ and R³ or R⁴ (as appropriate)) independently represent G¹ (e.g. halo (such as chloro), cyano, methyl, methoxy, trifluoromethyl or trifluoromethoxy) or, more preferably, hydrogen;
A represents G¹;
G¹ represents halo (e.g. chloro), cyano or, more preferably, -NO₂ or -A¹-R⁷;
A¹ represents a single bond, -N(R⁸)A⁴- or -OA⁵-;
A⁴ and A⁵ independently represent a single bond;
R⁷ and R⁸ are independently selected from hydrogen and C₁₋₆ alkyl, which latter group is optionally substituted by one or more substituents selected from G³;
G³ represents halo (especially fluoro).

Especially preferred compounds of the invention are wherein:
X represents a phenyl group, substituted, for example in the 3- or, preferably, 4-position, by a single -A¹-R⁷ group. In such instances, A¹ may represent -OA⁵-, in which A⁵ is as hereinbefore defined and preferably a single bond. R⁷ may, in such instances, represent C₁₋₄ alkyl, such as an optionally branched propyl, so forming, for example, a 4-isopropoxyphenyl group. Alternatively, X may represent a pyridyl group (e.g. a 3-pyridyl group), optionally substituted, for example at the *meta*- or, preferably, the *para*-position relative to the point of attachment of the X group to the indole ring, with a single -A¹-R⁷ group. In such instances A¹ may represent -OA⁵-in which A⁵ is as hereinbefore defined and, preferably, is a single bond and
R⁷ represents C₁₋₄ alkyl, such as an optionally branched propyl group, so forming, for example a 6-isopropoxypyrid-3-yl or 3-pyridyl group;
R⁶ represents hydrogen;
R¹ represents a naphthyl group (e.g. 2-naphthyl), preferably unsubstituted, or a phenyl group, substituted by an -A¹-R⁷ group and, optionally, a further -NO₂ group. In such instances A¹ may represent -OA⁵-, a single bond or -N(R⁸)A⁴-, in which A⁴ and A⁵ are as hereinbefore defined and are preferably single bonds. When A¹ represents -OA⁵-, R⁷ is preferably a C₁₋₄ alkyl group, such as an optionally branched propyl group or a methyl group, optionally substituted by one or more G³ group, in which G³ is preferably halo (especially fluoro). When A¹ represents a single bond, R⁷ is preferably a C₁₋₂ alkyl group, such as methyl. When A¹ represents -N(R⁸)A⁴-, R⁸ may represent H or C₁₋₂ alkyl, such as methyl, and R⁷ may represent C₁₋₄ alkyl, such as ethyl or, preferably, methyl or optionally branched propyl. Thus R¹ may represent isopropoxyphenyl (e.g. 2-, 3- and 4-isopropoxyphenyl), 4-dimethylaminophenyl, 4-isopropylaminophenyl, 4-trifluoromethoxyphenyl or 4-methyl-3-nitrophenyl;
   alternatively, R¹ may represent a pyridyl (e.g. a 2-pyridyl or 3-pyridyl) group, optionally substituted, for example by an -A¹-R⁷ group. In such instances, A¹ may represent -OA⁵- or a single bond, in which A⁵ is as hereinbefore defined and preferably a single bond. When A¹ represents -OA⁵-, R⁷ is preferably a C₁₋₄ alkyl group, for example a C₁₋₃ alkyl group such as an optionally branched propyl group. When A¹ represents a single bond, R⁷ is preferably a C₁₋₂ alkyl group, such as methyl, optionally substituted by one or more G³ group, in which G³ is as hereinbefore defined and preferably a fluoro group. Thus R¹ may also represent 5-trifluoromethylpyrid-2-yl, 6-isopropoxypyrid-3-yl or 3-pyridyl;
R² represents H;
R³ represents H, phenyl or pyridyl, which latter two groups are optionally substituted. For example, the phenyl group may be substituted in the 3- or, preferably, 4-position, by a single -A¹-R⁷ group. In such instances, A¹ may represent a single bond or -OA⁵-, wherein A⁵ is preferably a single bond, and R⁷ may represent C₁₋₄ alkyl, such as methyl or optionally branched butyl, optionally substituted by one or more G³ groups, in which G³ is halo, such as fluoro. Thus R³ may represent 4-*tert*-butylphenyl or 4-trifluoromethylphenyl;
R⁴ represents H, phenyl or pyridyl, which latter two groups are optionally substituted. For example, the phenyl group may be substituted in the 3- or, preferably, 4-position, by a single -A¹-R⁷ group. In such instances A¹ may represent
-OA⁵-, in which A⁵ is a single bond and R⁷ represents a C₁₋₄ alkyl group, preferably a C₁₋₃ alkyl group, such as an optionally branched propyl group, which alkyl group is optionally substituted by one or more G³ groups, in which G³ is halo, such as fluoro. Thus R⁴ may represent a 4-isopropoxyphenyl group;
R⁵ represents H.

Particularly preferred compounds of the invention include those of the examples described hereinafter.

Compounds of the invention may be made in accordance with techniques that are well known to those skilled in the art, for example as described hereinafter.

According to a further aspect of the invention there is provided a process for the preparation of a compound of formula I, which process comprises:
(i) reaction of a compound of formula II, 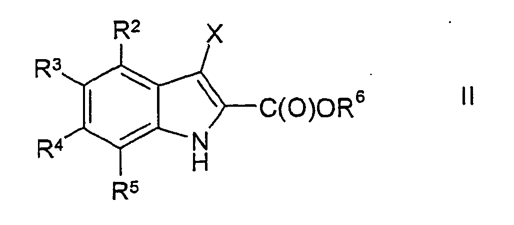 wherein X, R², R³, R⁴, R⁵ and R⁶ are as hereinbefore defined, with a compound of formula III,

   R¹L¹ III

   wherein L¹ represents a suitable leaving group such as chloro, bromo, iodo, a sulfonate group (e.g. -OS(O)₂CF₃, -OS(O)₂CH₃, -OS(O)₂PhMe or a nonaflate) or -B(OH)₂ and R¹ is as hereinbefore defined, for example optionally in the presence of an appropriate metal catalyst (or a salt or complex thereof) such as Cu, Cu(OAc)₂, CuI (or CuI/diamine complex), Pd(OAc)₂, Pd₂(dba)₃ or NiCl₂ and an optional additive such as Ph₃P, 2,2'-bis(diphenylphosphino)-1,1'-binaphthyl, xantphos, NaI or an appropriate crown ether such as 18-crown-6-benzene, in the presence of an appropriate base such as NaH, Et₃N, pyridine, *N*,*N*'-dimethylethylenediamine, Na₂CO₃, K₂CO₃, K₃PO₄, Cs₂CO₃, *t*-BuONa or *t*-BuOK (or a mixture thereof), in a suitable solvent (e.g. dichloromethane, dioxane, toluene, ethanol, isopropanol, dimethylformamide, ethylene glycol, ethylene glycol dimethyl ether, water, dimethylsulfoxide, acetonitrile, dimethylacetamide, N-methylpyrrolidinone, tetrahydrofuran or a mixture thereof) or in the absence of an additional solvent when the reagent may itself act as a solvent (e.g. when R¹ represents phenyl and L¹ represents bromo, i.e. bromobenzene). This reaction may be carried out at room temperature or above (e.g. at a high temperature, such as the reflux temperature of the solvent system that is employed) or using microwave irradiation;
(ii) reaction of a compound of formula IV, 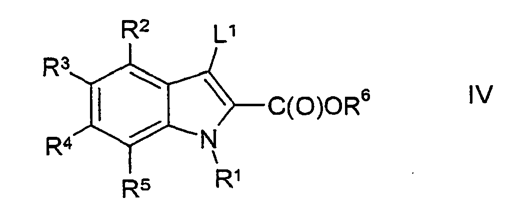 wherein L¹, R¹, R², R³, R⁴, R⁵ and R⁶ are as hereinbefore defined, with a compound of formula V,

   XL² V

   wherein L² represents a suitable leaving group such as chloro, bromo, iodo, -B(OH)₂ or a protected derivative thereof, for example a 4,4,5,5-tetramethyl-1,3,2-dioxaborolan-2-yl group, 9-borabicyclo[3.3.1]nonane (9-BBN), -Sn(alkyl)₃ (e.g. -SnMe₃ or -SnBu₃), or a similar group known to the skilled person, and X is as hereinbefore defined. The skilled person will appreciate that L¹ and L² will be mutually compatible. This reaction may be performed, for example in the presence of a suitable catalyst system, e.g. a metal (or a salt or complex thereof) such as CuI, PdCl₂, Pd/C, Pd(OAc)₂, Pd(Ph₃P)₂Cl₂, Pd(Ph₃P)₄, Pd₂(dba)₃ or NiCl₂ and an additive such as *t*-Bu₃P, (C₆H₁₁)₃P, Ph₃P, AsPh₃, P(*o*-Tol)₃, 1,2-bis(diphenylphosphino)ethane, 2,2'-bis(di-*tert*-butylphosphino)-1,1'-biphenyl, 2,2'-bis(diphenylphosphino)-1,1'-binaphthyl, 1,1'-bis(diphenylphosphinoferrocene), 1,3-bis(diphenylphosphino)propane or xantphos, together with a suitable base such as, Na₂CO₃, K₃PO₄, Cs₂CO₃, KOH, NaOH, K₂CO₃, CsF, Et₃N, (*i*-Pr)₂NEt, *t-*BuONa or *t*-BuOK (or mixtures thereof) in a suitable solvent such as dioxane, toluene, ethanol, dimethylformamide, ethylene glycol dimethyl ether, water, dimethylsulfoxide, acetonitrile, dimethylacetamide, *N-*methylpyrrolidinone, tetrahydrofuran or mixtures thereof. The reaction may also be carried out for example at room temperature or above (e.g. at a high temperature such as the reflux temperature of the solvent system) or using microwave irradiation. The skilled person will appreciate that when L¹ or L² (of the compounds of formulae IV and V, respectively, represent halo, such compounds may first be activated by:
   (I) forming the corresponding Grignard reagent under standard conditions known to those skilled in the art (e.g. employing magnesium or a suitable reagent such as a mixture of C₁₋₆ alkyl-Mg-halide and ZnCl₂ or LiCl), followed by reaction with a compound of formula IV or V (as appropriate), optionally in the presence of a catalyst (e.g. FeCl₃) under conditions known to those skilled in the art; or
   (II) forming the corresponding lithiated compound under halogen-lithium exchange reaction conditions known to those skilled in the art (e.g. employing *n*-BuLi or t-BuLi in the presence of a suitable solvent (e.g. a polar aprotic solvent, such as THF)), followed by reaction with a compound of formula IV or V (as appropriate).
   The skilled person will also appreciate that the magnesium of the Grignard reagent or the lithium of the lithiated species may be exchanged for a different metal (i.e. a transmetallation reaction may be performed), for example to zinc (e.g. using ZnCl₂) and the intermediate so formed may then be subjected to reaction with a compound of formula IV or V (as appropriate) under conditions known to those skilled in the art, for example such as those described above;
(iii) reaction of a compound of formula VI, 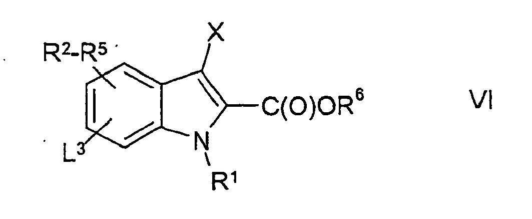 wherein L³ represents L¹ or L² as hereinbefore defined, which group is attached to one or more of the carbon atoms of the benzenoid ring of the indole, and the remaining positions of the benzenoid ring are substituted with 1 to 3 (depending on the number of L³ substituents) R²-R⁵ substituents, R²-R⁵ represents any one of the substituents, i.e. R², R³, R⁴ and R⁵, that are already present in that ring (as appropriate), and X, R¹, R², R³, R⁴, R⁵ and R⁶ are as hereinbefore defined, with a compound of formula VII,

   R¹⁷L⁴ VII

   wherein R¹⁷ represents R², R³, R⁴ or R⁵ (as appropriate), and L⁴ represents L¹ (when L³ is L²) or L² (when L³ is L¹) as hereinbefore defined. Such reactions may be performed for example under conditions such as those described hereinbefore in respect of process step (ii) above.

Compounds of formula II may be prepared by:
(a) reaction of a compound of formula VIII, 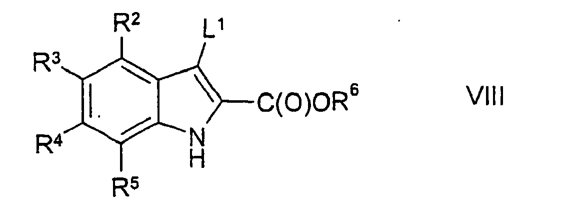 wherein L¹, R², R³, R⁴, R⁵ and R⁶ are as hereinbefore defined, with a compound of formula V as hereinbefore defined, for example under conditions such as those described hereinbefore in respect of preparation of compounds of formula I (process step (ii)) above;
(b) reaction of a compound of formula IX, 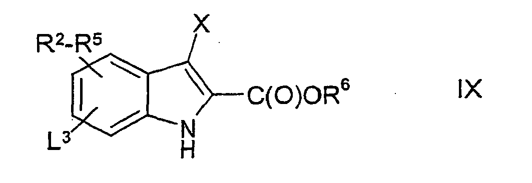 wherein X, L³, R²-R⁵ and R⁶ are as hereinbefore defined with a compound of formula VII as hereinbefore defined, for example under conditions such as those described hereinbefore in respect of preparation of compounds of formula I (process step (iii)) above.

Compounds of formula IV may be prepared by:
(a) reaction of a compound of formula VIII as hereinbefore defined with a compound of formula X,

   R¹L² X

   wherein R¹ and L² are as hereinbefore defined, for example under conditions such as those described hereinbefore in respect of preparation of compounds of formula I (process step (ii)) above;
(b) reaction of a compound of formula VIII as hereinbefore defined with a compound of formula III as hereinbefore defined, for example under reaction conditions such as those described hereinbefore in respect of preparation of compounds of formula I (process step (i)); or
(c) for compounds of formula IV wherein L¹ represents a sulfonate group, reaction of a compound of formula XI, 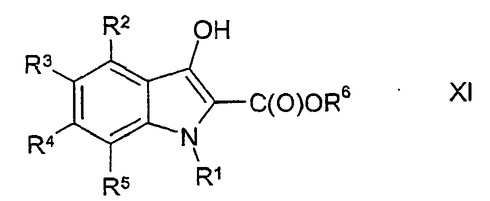 wherein R¹, R², R³, R⁴, R⁵ and R⁶ are as hereinbefore defined, with an appropriate reagent for the conversion of the hydroxyl group to the sulfonate group (e.g. tosyl chloride, mesyl chloride, triflic anhydride and the like) under conditions known to those skilled in the art.

Compounds of formula VI may be prepared by reaction of a compound of formula IX as hereinbefore defined, with a compound of formula III as hereinbefore defined, for example under reaction conditions such as those described hereinbefore in respect of preparation of compounds of formula I (process step (i)).

Compounds of formula VI in which L³ represents L² may be prepared by reaction of a compound of formula VI in which L³ represents L¹, with an appropriate reagent for the conversion of the L¹ group to the L² group. This conversion may be performed by methods known to those skilled in the art, for example:
i) compounds of formula VI, in which L³ is 4,4,5,5-tetramethyl-1,3,2-dioxaborolan-2-yl may be prepared by reaction of the reagent bis(pinacolato)diboron with a compound of formula VI in which L³ represents L¹, for example under reaction conditions such as those described hereinbefore in respect of preparation of compounds of formula I (process step (ii)) above;
ii) compounds of formula VI, in which L³ represents -B(OH)₂ may be prepared by reaction of a corresponding compound of formula VI in which L³ represents halo by reaction with, for example, boronic acid or a protected derivative thereof (e.g. bis(pinacolato)diboron or triethyl borate) followed by (if necessary) deprotection under standard conditions. The skilled person will appreciate that the compound of formula VI in which L³ represents halo may first need to be converted to the corresponding Grignard reagent, or another metal (e.g. *via* a transmetallation reaction), for example under conditions such as those described in respect of preparation of compounds of formula I (process step (ii)) above; or
iii) compounds of formula VI in which L³ represents a halo group may be prepared by reaction of a corresponding compound of formula VI in which L³ represents a different halo group, for example employing a suitable source of halide ions such as those described hereinafter in respect of preparation of compounds of formula VIII (process (a)) under conditions known to those skilled in the art. For example, conversion of a bromo group to an iodo group may be performed in the presence of NaI, optionally in the presence of a suitable catalyst (e.g. CuI) and/or a catalytic amount of base (e.g. *N*'*N*,-dimethyl-1,2-diaminoethane) in the presence of a suitable solvent such as one described hereinbefore in respect of preparation of compounds of formula I (process step (i)).

Conversions of the L¹, L⁴ group and the L³ group in the compounds of formulae IV, VII and IX, respectively, may be performed in a similar manner to that described above in respect of converting the L³ group in compounds of formula VI.

Compounds of formula VIII may be prepared by standard techniques. For example:
(a) compounds of formula VIII, wherein L¹ represents bromo or iodo, may be prepared by reaction of a compound of formula XII, 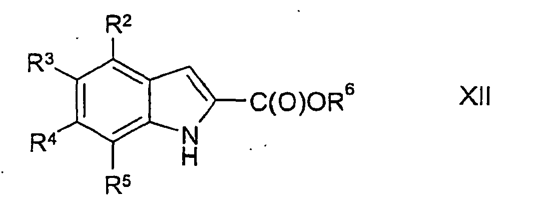 wherein R², R³, R⁴, R⁵ and R⁶ are as hereinbefore defined, with a reagent, or mixture of reagents known to be a source of bromide or iodide ions (e.g. *N*-bromosuccinimide, iodine, or a mixture of NaI and *N*-chlorosuccinimide). This reaction may be carried out, for example, at room temperature in a suitable solvent (e.g. acetone or benzene);
(b) by reaction of a compound of formula XIII, 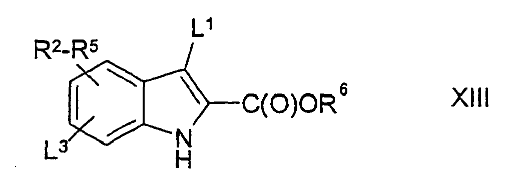 wherein L¹, L³, R²-R⁵ and R⁶ are as hereinbefore defined with a compound of formula VII as hereinbefore defined, for example under reaction conditions such as those described hereinbefore in respect of preparation of compounds of formula I (process step (iii)) above; or
(c) compounds of formula VIII, wherein L¹ represents a sulfonate group may be prepared by reaction of a compound of formula XIV, 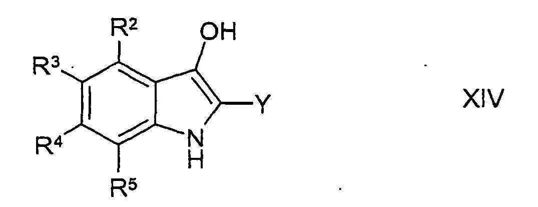 wherein Y, R², R³, R⁴ and R⁵ are as hereinbefore defined, with an appropriate reagent for the conversion of the hydroxyl group to a sulfonate group as described hereinbefore.

Compounds of formulae III, V, VII, IX, X, XI, XII, XIII and XIV are either commercially available, are known in the literature, or may be obtained either by analogy with the processes described herein, or by conventional synthetic procedures, in accordance with standard techniques, from available starting materials using appropriate reagents and reaction conditions. In this respect, the skilled person may refer to *inter alia* "Comprehensive Organic Synthesis" by B. M. Trost and I. Fleming, Pergamon Press, 1991.

Indoles of formulae II, IV, VI, VIII IX, XI, XII, XIII and XIV may also be prepared with reference to a standard heterocyclic chemistry textbook (e.g. "Heterocyclic Chemistry" by J. A. Joule, K. Mills and G. F. Smith, 3rd edition, published by Chapman & Hall or "Comprehensive Heterocyclic Chemistry II" by A. R. Katritzky, C. W. Rees and E. F. V. Scriven, Pergamon Press, 1996) and/or made according to the following general procedures.

For example, compounds of formulae II, IX and XII may be prepared by reaction of a compound of formula XV, 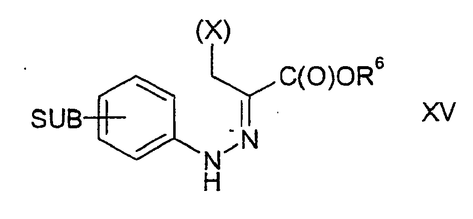 wherein SUB represents the substitution pattern that is present in the compound of formula II, IX or XII to be formed, (X) either represents the substituent X as hereinbefore defined (as required for formation of compounds of formula II and IX) or hydrogen (as required for formation of compounds of formula XII) and R⁶ is as hereinbefore defined, under Fischer indole synthesis conditions known to the person skilled in the art.

Compounds of formula XII may alternatively be prepared by reaction of a compound of formula XVI, 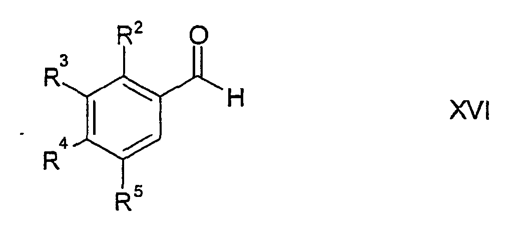 wherein R², R³, R⁴ and R⁵ are as hereinbefore defined with a compound of formula XVII,

N₃CH₂C(O)OR⁶ XVII

wherein R⁶ is as hereinbefore defined, and preferably does not represent hydrogen, under conditions known to the person skilled in the art (i.e. conditions to induce a condensation reaction, followed by a thermally induced cyclisation).

Compounds of formulae XI and XIV may be prepared by reaction of a compound of formula XVIII, 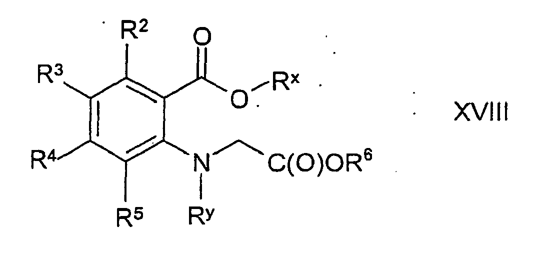 wherein R^{x} represents a C₁₋₆ alkyl group, R^{y} represents either R¹ as hereinbefore defined (as required for formation of compounds of formula XI) or hydrogen (as required for formation of compounds of formula XIV), or a nitrogen-protected derivative thereof, and R², R³, R⁴, R⁵ and R⁶ are as hereinbefore defined for example under cyclisation conditions known to those skilled in the art.

Compounds of formula II and IX may alternatively be prepared by reaction of a compound of formula XIX, 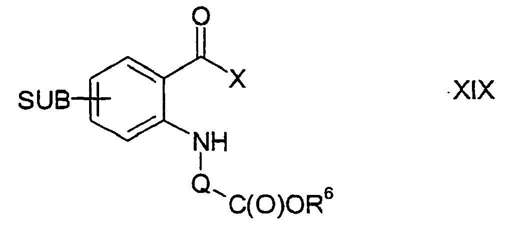 wherein Q represents either -C(O)- or -CH₂- and SUB, X and R⁶ are as hereinbefore defined. When Q represents -C(O)-, the intramolecular cyclisation may be induced by a reducing agent such as TiCl₃/C₈K, TiCl₄/Zn or SmI₂ under conditions known to the skilled person, for example, at room temperature in the presence of a polar aprotic solvent (such as THF). When Q represents -CH₂-, the reaction may be performed in the presence of base under intramolecular condensation reaction conditions known to the skilled person.

Compounds of formula XV may be prepared by:
(a) reaction of a compound of formula XX, 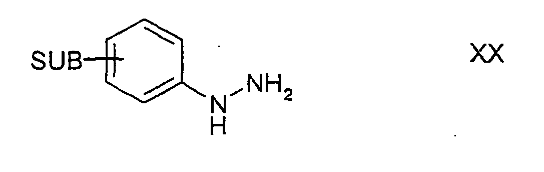 wherein SUB is as hereinbefore defined with a compound of formula XXI, 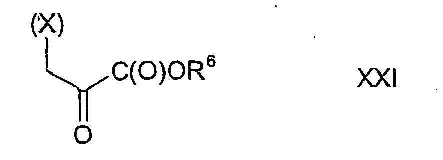 wherein (X) and R⁶ are as hereinbefore defined under condensation conditions known to the skilled person; or
(b) reaction of a compound of formula XXII, 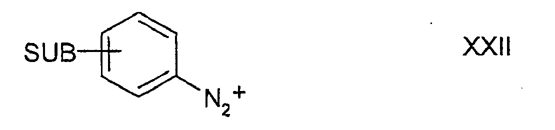 wherein SUB is as hereinbefore defined with a compound of formula XXIII, 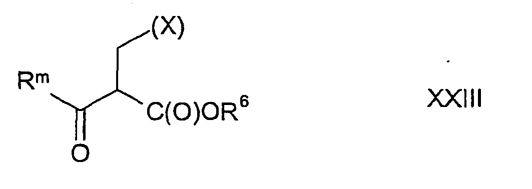 wherein R^{m} represents OH, O-C₁₋₆ alkyl or C₁₋₆ alkyl and (X) and R⁶ are as hereinbefore defined, for example under Japp-Klingemann conditions known to the skilled person.

Compounds of formulae XVI, XVII, XVIII, XIX, XX, XXI, XXII and XXIII are either commercially available, are known in the literature, or may be obtained either by analogy with the processes described herein, or by conventional synthetic procedures, in accordance with standard techniques, from available starting materials using appropriate reagents and reaction conditions. In this respect, the skilled person may refer to *inter alia* "Comprehensive Organic Synthesis" by B. M. Trost and I. Fleming, Pergamon Press, 1991.

The substituents X, R¹, R², R³, R⁴, R⁵ and R⁶ in final compounds of the invention or relevant intermediates may be modified one or more times, after or during the processes described above by way of methods that are well known to those skilled in the art. Examples of such methods include substitutions, reductions, oxidations, alkylations, hydrolyses, esterifications, and etherifications. The precursor groups can be changed to a different such group, or to the groups defined in formula I, at any time during the reaction sequence. For example, in cases where R⁶ does not initially represent hydrogen (so providing an ester functional group), the skilled person will appreciate that at any stage during the synthesis (e.g. the final step), the relevant substituent may be hydrolysed to form a carboxylic acid functional group (in which case R⁶ will be hydrogen). In this respect, the skilled person may also refer to "Comprehensive Organic Functional Group Transformations" by A. R. Katritzky, O. Meth-Cohn and C. W. Rees, Pergamon Press, 1995.

Compounds of the invention may be isolated from their reaction mixtures using.conventional techniques.

It will be appreciated by those skilled in the art that, in the processes described above and hereinafter, the functional groups of intermediate compounds may need to be protected by protecting groups.

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described herein may be converted chemically to unprotected compounds using standard deprotection techniques.

The type of chemistry involved will dictate the need, and type, of protecting groups as well as the sequence for accomplishing the synthesis.

The use of protecting groups is fully described in "protective Groups in Organic Chemistry", edited by J W F McOmie, Plenum Press (1973), and "Protective Groups in Organic Synthesis", 3rd edition, T.W. Greene & P.G.M. Wutz, Wiley-Interscience (1999).

### Medical and Pharmaceutical Uses

Compounds of the invention are indicated as pharmaceuticals. According to a further aspect of the invention there is provided a compound of the invention for use as a pharmaceutical.

Although compounds of the invention may possess pharmacological activity as such, certain pharmaceutically-acceptable (e.g. "protected") derivatives of compounds of the invention may exist or be prepared which may not possess such activity, but may be administered parenterally or orally and thereafter be metabolised in the body to form compounds of the invention. Such compounds (which may possess some pharmacological activity, provided that such activity is appreciably lower than that of the "active" compounds to which they are metabolised) may therefore be described as "prodrugs" of compounds of the invention.

By "prodrug of a compound of the invention", we include compounds that form a compound of the invention, in an experimentally-detectable amount, within a predetermined time (e.g. about 1 hour), following oral or parenteral administration. All prodrugs of the compounds of the invention are included within the scope of the invention.

Furthermore, certain compounds of the invention (including, but not limited to, compounds of formula I in which R⁶ is other than hydrogen) may possess no or minimal pharmacological activity as such, but may be administered parenterally or orally, and thereafter be metabolised in the body to form compounds of the invention that possess pharmacological activity as such (including, but not limited to, corresponding compounds of formula I, in which R⁶ represents hydrogen). Such compounds (which also includes compounds that may possess some pharmacological activity, but that activity is appreciably lower than that of the "active" compounds of the invention to which they are metabolised), may also be described as "prodrugs".

Thus, the compounds of the invention are useful because they possess pharmacological activity, and/or are metabolised in the body following oral or parenteral administration to form compounds which possess pharmacological activity.

Compounds of the invention are particularly useful because they may inhibit (for example selectively) the activity of prostaglandin E synthases (and particularly microsomal prostaglandin E synthase-1 (mPGES-1)), i.e. they prevent the action of mPGES-1 or a complex of which the mPGES-1 enzyme forms a part, and/or may elicit a mPGES-1 modulating effect, for example as may be demonstrated in the test described below. Compounds of the invention may thus be useful in the treatment of those conditions in which inhibition of a PGES, and particularly mPGES-1, is required.

Compounds of the invention may inhibit the activity of leukotriene C₄ (LTC₄) synthase, for example as may be shown in a test such as that described in Eur. J. Biochem., 208, 725-734 (1992), and may thus be useful in the treatment of those conditions in which inhibition of LTC₄ synthase is required. Compounds of the invention may also inhibit the activity of 5-lipoxygenase-activating protein (FLAP), for example as may be shown in a test such as that described in Mol. Pharmacol., 41, 873-879 (1992).

Compounds of the invention are thus expected to be useful in the treatment of inflammation.

The term "inflammation" will be understood by those skilled in the art to include any condition characterised by a localised or a systemic protective response, which may be elicited by physical trauma, infection, chronic diseases, such as those mentioned hereinbefore, and/or chemical and/or physiological reactions to external stimuli (e.g. as part of an allergic response). Any such response, which may serve to destroy, dilute or sequester both the injurious agent and the injured tissue, may be manifest by, for example, heat, swelling, pain, redness, dilation of blood vessels and/or increased blood flow, invasion of the affected area by white blood cells, loss of function and/or any other symptoms known to be associated with inflammatory conditions.

The term "inflammation" will thus also be understood to include any inflammatory disease, disorder or condition *per se*, any condition that has an inflammatory component associated with it, and/or any condition characterised by inflammation as a symptom, including *inter alia* acute, chronic, ulcerative, specific, allergic and necrotic inflammation, and other forms of inflammation known to those skilled in the art. The term thus also includes, for the purposes of this invention, inflammatory pain, pain generally and/or fever.

Accordingly, compounds of the invention may be useful in the treatment of inflammatory bowel disease, irritable bowel syndrome, migraine, headache, low back pain, fibromyalgia, myofascial disorders, viral infections (*e*.*g*. hepatitis C and, particularly, influenza, common cold, herpes zoster, and AIDS), bacterial infections, fungal infections, dysmenorrhea, bums, surgical or dental procedures, malignancies (*e*.*g*. breast cancer, colon cancer, and prostate cancer), atherosclerosis, gout, arthritis, osteoarthritis, juvenile arthritis, rheumatoid arthritis, fever (e.g. rheumatic fever), ankylosing spondylitis, systemic lupus erythematosus, vasculitis, pancreatitis, nephritis, bursitis, conjunctivitis, iritis, scleritis, uveitis, wound healing, dermatitis, eczema, psoriasis, stroke, diabetes mellitus, neurodegenerative disorders such as Alzheimer's disease and multiple sclerosis, autoimmune diseases, osteoporosis, asthma, chronic obstructive pulmonary disease, pulmonary fibrosis, allergic disorders, rhinitis, ulcers, coronary heart disease, sarcoidosis and any other disease with an inflammatory component. Other diseases that may be mentioned include inflammatory pain, hyperprostaglandin E syndrome, classic Bartter syndrome, Hodgkin's disease and persistent ductus (PDA).

Compounds of the invention may also have effects that are not linked to inflammatory mechanisms, such as in the reduction of bone loss in a subject. Conditions that may be mentioned in this regard include osteoporosis, osteoarthritis, Paget's disease and/or periodontal diseases. Compounds the invention may thus also be useful in increasing bone mineral density, as well as the reduction in incidence and/or healing of fractures, in subjects.

Compounds of the invention are indicated both in the therapeutic and/or prophylactic treatment of the above-mentioned conditions.

According to a further aspect of the present invention, there is provided the use of a compound of the invention as hereinbefore defined for the manufacture of a medicament for a method of treatment of a disease which is associated with, and/or which can be modulated by inhibition of LTC₄ synthase, FLAP and/or, preferably, a PGES (such as mPGES-1), and/or a method of treatment of a disease in which inhibition of the activity of LTC₄ synthase, FLAP and/or, preferably, a PGES (and particularly mPGES-1) is desired and/or required (e.g. inflammation), which method comprises administration of a therapeutically effective amount of such a compound to a patient suffering from, or susceptible to, such a condition.

"Patients" include mammalian (including human) patients.

The term "effective amount" refers to an amount of a compound, which confers a therapeutic effect on the treated patient. The effect may be objective (i.e. measurable by some test or marker) or subjective (i.e. the subject gives an indication of or feels an effect).

Compounds of the invention will normally be administered orally, intravenously, subcutaneously, buccally, rectally, dermally, nasally, tracheally, bronchially, sublingually, by any other parenteral route or *via* inhalation, in a pharmaceutically acceptable dosage form.

Compounds of the invention may be administered alone, but are preferably administered by way of known pharmaceutical formulations, including tablets, capsules or elixirs for oral administration, suppositories for rectal administration, sterile solutions or suspensions for parenteral or intramuscular administration, and the like.

Such formulations may be prepared in accordance with standard and/or accepted pharmaceutical practice.

According to a further aspect of the invention there is thus provided a pharmaceutical formulation including a compound of the invention, as hereinbefore defined, in admixture with a pharmaceutically acceptable adjuvant, diluent or carrier.

Compounds of the invention may also be combined with other therapeutic agents that are useful in the treatment of inflammation (e.g. NSAIDs and coxibs).

According to a further aspect of the invention, there is provided a combination product comprising:
(A) a compound of the invention, as hereinbefore defined; and
(B) another therapeutic agent that is useful in the treatment of inflammation,
wherein each of components (A) and (B) is formulated in admixture with a pharmaceutically-acceptable adjuvant, diluent or carrier.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent that is useful in the treatment of inflammation, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of inflammation in admixture with a pharmaceutically-acceptable adjuvant, diluent or carrier,
   which components (a) and (b) are each provided in a form that is suitable for administration in conjunction with the other.

Compounds of the invention may be administered at varying doses. Oral, pulmonary and topical dosages may range from between about 0.01 mg/kg of body weight per day (mg/kg/day) to about 100 mg/kg/day, preferably about 0.01 to about 10 mg/kg/day, and more preferably about 0.1 to about 5.0 mg/kg/day. For e.g. oral administration, the compositions typically contain between about 0.01 mg to about 500 mg, and preferably between about 1 mg to about 100 mg, of the active ingredient. Intravenously, the most preferred doses will range from about 0.001 to about 10 mg/kg/hour during constant rate infusion. Advantageously, compounds may be administered in a single daily dose, or the total daily dosage may be administered in divided doses of two, three or four times daily.

In any event, the physician, or the skilled person, will be able to determine the actual dosage which will be most suitable for an individual patient, which is likely to vary with the route of administration, the type and severity of the condition that is to be treated, as well as the species, age, weight, sex, renal function, hepatic function and response of the particular patient to be treated. The above-mentioned dosages are exemplary of the average case; there can, of course, be individual instances where higher or lower dosage ranges are merited, and such are within the scope of this invention.

Compounds of the invention may have the advantage that they are effective, and preferably selective, inhibitors of prostaglandin E synthases (PGES) and particularly microsomal prostaglandin E synthase-1 (mPGES-1). The compounds of the invention may reduce the formation of the specific arachidonic acid metabolite PGE₂ without reducing the formation of other COX generated arachidonic acid metabolites, and thus may not give rise to the associated side-effects mentioned hereinbefore.

Compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise.

### Biological Test

In the assay human mPGES-1 catalyses the reaction where the substrate PGH₂ is converted to PGE₂. mPGES-1 is expressed in *E. coli* and the membrane fraction is dissolved in 20mM NaPi-buffer pH 8.0 and stored at -80 °C. In the assay human mPGES-1 is dissolved in 0.1 M KPi-buffer pH 7.35 with 2.5 mM glutathione. The stop solution consists of H₂O / MeCN (7/3), containing FeCl₂ (25 mM) and HCl (0.15 M). The assay is performed at room temperature in 96-well plates. Analysis of the amount of PGE₂ is performed with reversed phase HPLC (Waters 2795 equipped with a 3.9 x 150 mm C18 column). The mobile phase consists of H₂O / MeCN (7/3), containing TFA (0.056%), and absorbance is measured at 195 nm with a Waters 2487 UV-detector.
The following is added chronologically to each well:
1. 100 µL human mPGES-1 in KPi-buffer with glutathione. Total protein concentration: 0.02 mg/mL.
2. 1 µL inhibitor in DMSO. Incubation of the plate at room temperature for 25 minutes.
3. 4 µL of a 0.25 mM PGH₂ solution. Incubation of the plate at room temperature for 60 seconds.
4. 100 µL stop solution. 180 µL per sample is analyzed with HPLC.

### Examples

The invention is illustrated by way of the following examples, in which the following abbreviations may be employed:
- cy: cyclohexyl
- dba: dibenzylideneacetone
- DIBAL: diisobutylaluminium hydride
- DMAP: 4,4-dimethylaminopyridine
- DMF: dimethylformamide
- DMSO: dimethylsulfoxide
- EtOAc: ethyl acetate
- HPLC: High Pressure Liquid Chromatography
- MeCN: acetonitrile
- MS: mass spectrum
- NMR: nuclear magnetic resonance
- TFA: trifluoroacetic acid
- THF: tetrahydrofuran
- xantphos: 9,9-dimethyl-4,5-bis(diphenylphosphino)-xanthene

Starting materials and chemical reagents specified in the syntheses described below are commercially available from, e.g. Sigma-Aldrich Fine Chemicals.

### Example 1

### 5-(4-tert-Butylphenyl)-1,3-bis(4-isopropoxyphenyl)-indole-2-carboxylic acid

### (a) 5-(4-tert-Butylphenyl)indole-2-carboxulic acid ethyl ester

A mixture of 5-bromoindole-2-carboxylic acid ethyl ester (3.48 g, 13 mmol), 4-*tert*-butylphenylboronic acid (4.63 g, 26 mmol), K₃PO₄ (9.93 g, 45 mmol), Pd(OAc)₂ (146 mg, 0.65 mmol), tri-o-tolylphosphine (396 mg, 1.3 mmol), EtOH (20 ml) and toluene (10 mL) was stirred under argon for 20 min at room temperature, and then heated at 100°C for 24 h. The mixture was allowed to cool, poured into NaHCO₃ (aq., sat.) and extracted with EtOAc. The combined extracts were washed with water and brine and then dried over Na₂SO₄. Concentration and purification by chromatography gave the sub-title compound (3.27 g, 78%).

### (b) 5-(4-tert-Butylphenyl)-3-iodoindole-2-carboxylic acid ethyl ester

This reaction was performed with the exclusion of light. A solution of NaI (300mg, 2.0 mmol) in acetone (15 mL) was added dropwise to a stirred solution of *N*-chlorosuccinimide (270 mg, 2.0 mmol) in acetone (4 mL), followed, after 15 min, by the dropwise addition of 5-(4-*tert-*butylphenyl)indole-2-carboxylic acid ethyl ester (650mg, 2.0 mmol; see step (a) above) in acetone (20 mL). After 30 min at room temperature the mixture was poured into Na₂S₂O₃ (aq., 10%, 40 mL) and extracted with EtOAc. The combined extracts were washed with water and brine and then dried over Na₂SO₄. The organic phase was then concentrated and purified by chromatography to give the sub-title compound. This product was employed in the subsequent steps without further purification.

### (c) 5-(4-tert-Butylpheny)-3-(4-isopropoxyphenyl)-indole-2-carboxylic acid ethyl ester

5-(4-*tert*-Butylphenyl)-3-iodoindole-2-carboxylic acid ethyl ester (146 mg, 0.32 mmol; see step (b) above), 4-isopropoxyphenylboronic acid (86 mg, 0.48 mmol), K₃PO₄ (238 mg, 1.12 mmol), Pd(OAc)₂ (3.6 mg, 0.016 mmol) and toluene (3 mL) was stirred for 20 min at room temperature and for 4 h at 80°C. The mixture was poured into NaHCO₃ (aq., sat.) and extracted with EtOAc. The combined extracts were washed with water and brine and then dried over Na₂SO₄. Concentration and purification by column chromatography gave the sub-title compound.

### (d) 5-(4-tert-Butylphenyl)-1,3-bis(4-isopropoxyphenyl)-indole-2-carboxylic acid ethyl ester

Anhydrous CH₂Cl₂ (15 mL), followed by triethylamine (490 µL, 352 mg, 3.48 mmol), pyridine (280 µL, 275 mg, 3.48 mmol) and 3Å molecular sieves (ca. 2 g) were added to 5-(4-*tert*-butylphenyl)-3-(4-isopropoxyphenyl)indole-2-carboxylic acid ethyl ester (793 mg, 1.74 mmol; see step (c) above), Cu(OAc)₂ (632 mg, 3.48 mmol), and 4-isopropoxyphenylboronic acid (626 mg, 3.48 mmol). The mixture was stirred vigorously at ambient temperature for 30 h and was then filtered through Celite^{®}. The filter cake was washed with EtOAc, concentrated and purified by chromatography to afford the sub-title compound.

### (e) 5-(4-tert-Butylphenyl)-1,3-bis(4-isopropoxyphenyl)-indole-2-carboxylic acid

A mixture of 5-(4-*tert*-butylphenyl)-1,3-bis(4-isopropoxyphenyl)-indole-2-carboxylic acid ethyl ester (166 mg, 0.281 mmol; see step (d) above), aqueous NaOH (1M, 10 mL) and MeCN (40 mL) was heated at reflux for 4 h, allowed to cool, acidified with 1M HCl to pH 2 and extracted with EtOAc. The combined extracts were washed with water and brine and then dried over Na₂SO₄. Concentration, purification by chromatography, and successive recrystallisations from EtOH and then MeCN gave the title compound.
200 MHz ¹H-NMR (DMSO-d₆, ppm) δ 13.1-12.5 (1H, br s), 7.69 (1H, s), 7.59-7.37 (7H, m), 7.36-7.25 (2H, m), 7.19-7.11 (1H, m), 7.10-6.94 (4H, m), 4.67 (1H, septet, J=6.1 Hz), 4.65 (1H, septet J=6.1 Hz), 1.31 (6H, d, J=6.1 Hz), 1.30 (6H, d, J=6.1 Hz), 1.27 (9H, s).

### Example 2

### 5-(4-tert-Butylphenyl)-1-(3-isopropoxyphenyl)-3-(4-isoproxyphenyl)-indole-2-carboxylic acid

The title compound was prepared in accordance with Example 1, using 5-bromoindole-2-carboxylic acid ethyl ester, 4-*tert*-butylphenylboronic acid, 4-isopropoxyphenylboronic acid, and 3-isopropoxyphenylboronic acid. 200 MHz ¹H-NMR (DMSO-d₆, ppm) δ 12.9-12.8 (1H, br s), 7.72-7.70 (1H, m), 7.62-7.35 (8H, m), 7.30-7.21 (1H, m), 7.07-6.90 (5H, m), 4.77-4.55 (2H, m), 1.38-1.23 (21H, m).

### Example 3

### 5-(4-tert-Butylphenyl)-1-(2-isopropoxyphenyl)-3-(4-isopropoxyphenyl)-indole-2-carboxylic acid

The title compound was prepared in accordance with Example 1, using 5-bromoindole-2-carboxylic acid ethyl ester, 4-*tert*-butylphenylboronic acid, 4-isopropoxyphenylboronic acid and 2-isopropoxyphenylboronic acid. 200 MHz ¹H-NMR (DMSO-d₆, ppm) δ 12.5-12.4 (1H, br s), 7.65 (1H, s), 7.58-7.33 (9H, m), 7.24-6.93 (5H, m), 4.66 (1H, septet, J=6.0 Hz), 4.46 (1H, septet, J=6.0 Hz), 1.31 (6H, d, J=6.0 Hz), 1.28 (9H, s), 1.10 (3H, d, J=6.0 Hz), 1.02 (3H, d, J=6.0 Hz).

### Example 4

### 5-(4-tert-Butylphenyl)-3-(4-isopropoxyphenyl)-1-(5-(trifluoromethyl)-pyrid-2-yl)indole-2-carboxylic acid

### (a) 5-(4-tert-Butylphenyl)-3-(4-isopropoxyphenyl)-1-(5-(trifluoromethyl)-pyrid-2-yl)indole-2-carboxylic acid ethyl ester

CuI (7.6 mg, 40 nmol), *N*,*N*'-dimethyl-1,2-diaminoethane (13 µL, 120 nmol) and toluene (0.5 mL) were added to a mixture of 5-(4-*tert*-butylphenyl)-3-(4-isopropoxyphenyl)indole-2-carboxylic acid ethyl ester (182 mg, 400 nmol; see Example 1(c)), 2-bromo-5-(trifluoromethyl)pyridine (181 mg, 800 mmol), K₃PO₄ (196 mg, 800 nmol) and toluene (2 mL) under argon. The mixture was heated at 110°C for 27 h. Additional portions of CuI (7.6 mg, 40 nmol) and *N*,*N*'-dimethyl-1,2-diaminoethane (13 µL, 120 nmol) were added and the heating was continued for a further 22 h. The mixture was filtered through Celite^{®} and the filter cake was washed with EtOAc. The filtrate was concentrated and purified by chromatography to give the sub-title compound (66 mg, 28%).

### (b) 5-(4-tert-Butylphenyl)-3-(4-isopropoxyphenyl)-1-(5-(trifluoromethyl)-pyrid-2-yl)indole-2-carboxylic acid

The title compound was prepared by hydrolysis of 5-(4-*tert*-butylphenyl)-3-(4-isopropoxyphenyl)-1-(5-(trifluoromethyl)pyrid-2-yl)indole-2-carboxylic acid ethyl ester (see step (a) above) in accordance with the procedure described in Example 1(e).
200 MHz ¹H-NMR (DMSO-d₆, ppm) δ 13.2-13.0 (1H, br s), 9.03 (1H, s), 8.47 (1H, dd, J=8.6, 2.2 Hz), 7.90 (1H, d, J=8.6 Hz), 7.75-7.65 (3H, m), 7.60-7.40 (6H, m), 7.09-6.98 (2H, m), 4.68 (1H, septet, J=6.0 Hz), 1.31 (6H, d, J=6.0 Hz), 1.28 (9H, s).

### Example 5

### 5-(4-tert-Butylphenyl)-1-(4-(dimethylamino)phenyl)-3-(4-isopropoxyphenyl)indole-2-carboxylic acid

The title compound was prepared in accordance with Example 4, using 5-bromoindole-2-carboxylic acid ethyl ester, 4-*tert*-butylphenylboronic acid, 4-isopropoxyphenylboronic acid, and 1-bromo-4-(dimethylamino)benzene. 200 MHz ¹H-NMR (DMSO-d₆, ppm) δ 12.9-12.8 (1H, br s), 7.78 (1H, s), 7.66-7.45 (7H, m), 7.35-7.19 (3H, m), 7.14-7.02 (2H, m), 6.99-6.85 (2H, m), 4.74 (1H, septet, J=6.0 Hz), 3.06 (6H, s), 1.39 (6H, d, J=6.0 Hz), 1.37 (9H, s).

### Example 6

### 5-(4-tert-Butylphenyl)-3-(4-isopropoxyphenyl)-1-(4-(isopropylamino)-phenyl)indole-2-carboxylic acid

The title compound was prepared in accordance with Example 4, using 5-bromoindole-2-carboxylic acid ethyl ester, 4-*tert*-butylphenylboronic acid, 4-isopropoxyphenylboronic acid, and 1-bromo-4-(isopropylamino)-benzene.
200 MHz ¹H-NMR (DMSO-d₆, ppm) δ 7.70 (1H, s), 7.62-7.36 (7H, m), 7.19-7.05 (3H, m), 7.04-6.92 (2H, m), 6.70-6.58 (2H, m), 5.8-5.6 (1H, br s), 4.65 (1H, septet, J=6.1 Hz), 3.65-3.47 (1H, m), 1.30 (6H, d, J=6.1 Hz), 1.28 (9H, s), 1.17 (6H, d, J=6.3 Hz).

### Example 6A

### 5-(4-tert-Butylphenyl)-3-(4-isopropoxyphenyl)-1-(4-(isopropylamino)-phenyl)indole-2-carboxylic acid hydrochloride

4M HCl in dioxane (1.5 eq.) was added to 5-(4-*tert*-butylphenyl)-3-(4-isopropoxyphenyl)-1-(4-(isopropylamino)phenyl)indole-2-carboxylic acid (0.1 mmol/mL; see Example 6) in anhydrous Et₂O. The solvent was removed under reduced pressure and the residue triturated with anhydrous Et₂O. The solid was collected by filtration and dried *in vacuo*.
200 MHz ¹H-NMR (DMSO-d₆, ppm) δ 7.69 (1H, s), 7.64-6.94 (14H, m), 4.66 (1H, septet, J=6.1 Hz), 3.54-3.55 (1H, m), 1.30 (6H, d, J=6.1 Hz), 1.28 (9H, s), 1.22 (6H, d, J=6.3 Hz).

### Example 7

### 5-(4-tert-Butylphenyl)-3-(4-isopropoxyphenyl)-1-(6-isopropoxypyrid-3-yl)indole-2-carboxylic acid

The title compound was prepared in accordance with Example 4, using 5-bromoindole-2-carboxylic acid ethyl ester, 4-*tert*-butylphenylboronic acid, 4-isopropoxyphenylboronic acid, and 5-bromo-2-isopropoxypyridine.
200 MHz ¹H-NMR (DMSO-d₆, ppm) δ 13.0-12.8 (1H, br s), 8.23 (1H, d, J=2.7 Hz), 7.79 (1H, dd, J=8.7, 2.7 Hz), 7.68 (1H, s), 7.58-7.37 (7H, m), 7.14 (1H, d, J=8.7 Hz), 7.04-6.94 (2H, m), 6.89 (1H, d, J=8.8 Hz), 5.25 (1H, septet, J=6.2 Hz), 4.65 (1H, septet, J=6.0 Hz), 1.34 (6H, d, J=6.2 Hz), 1.30 (6H, d, J=6.0 Hz), 1.28 (9H, s).

### Example 7A

### 5-(4-tert-Butylphenyl)-3-(4-isopropoxyphenyl)-1-(6-isopropoxypyrid-3-yl)indole-2-carboxylic acid hydrochloride

The title compound was prepared in accordance with Example 6A from 5-(4-*tert*-Butylphenyl)-3-(4-isopropoxyphenyl)-1-(6-isopropoxypyrid-3-yl)-indole-2-carboxylic acid (see Example 7).
200 MHz ¹H-NMR (DMSO-d₆, ppm) δ 8.23 (1H, d, J=2.7 Hz), 7.79 (1H, dd, J=8.7, 2.7 Hz), 7.67 (1H, s), 7.62-7.34 (7H, m), 7.15 (1H, d, J=8.7 Hz), 7.05-6.96 (2H, m), 6.91 (1H, d, J=8.7 Hz), 5.30 (1H, septet, J=6.2 Hz), 4.66 (1H, septet, J=6.0 Hz), 1.34 (6H, d, J=6.2 Hz), 1.30 (6H, d, J=6.0 Hz), 1.28 (9H, s).

### Example 8

### 5-(4-tert-Butylphenyl)-3-(4-isopropoxyphenyl)-1-(4-methyl-3-nitrophenyl)indole-2-carboxylic acid

The title compound was prepared in accordance with Example 4, using 5-bromoindole-2-carboxylic acid ethyl ester, 4-*tert*-butylphenylboronic acid, 4-isopropoxyphenylboronic acid, and 4-bromo-1-methyl-2-nitrobenzene. 200 MHz ¹H-NMR (DMSO-d₆, ppm) δ 13.0 (1H, br s), 8.10 (1H, d, J=2.0 Hz), 7.76 (1H, dd, J=8.2, 2.0 Hz), 7.72-7.65 (2H, m), 7.61 (1H, dd, J=8.7, 1.4 Hz), 7.56-7.38 (6H, m), 7.25 (1H, d, J=8.7 Hz), 7.06-6.96 (2H, m), 4.67 (1H, septet, J=6.0 Hz) 2.61 (3H, s) 1.31 (6H, d, J=6.0 Hz) 1.30 (9H, s).

### Example 9

### 3.6-Bis(4-isopropoxyphenyl)-1-(naphthalen-2-yl)indole-2-carboxylic acid

The title compound was prepared in accordance with Example 1, using 6-bromoindole-2-carboxylic acid ethyl ester, 4-isopropoxyphenylboronic acid, and naphthalen-2-ylboronic acid.
200 MHz ¹H-NMR (DMSO-d₆, ppm) δ 8.12-7.95 (4H, m), 7.70-7.36 (9H, m), 7.33-7.28 (1H, m), 7.05-6.84 (4H, m), 4.64 (1H, septet, J= 6.0 Hz), 4.56 (1H, septet, J= 6.0 Hz), 1.30 (6H, d, J=6.0 Hz), 1.22 (6H, d, J=6.0 Hz).

### Example 9A

### Sodium 3,6-bis(4-isopropoxyphenyl)-1-(naphthalen-2-yl)indole-2-carboxylate

To a solution of 3,6-bis(4-isopropoxyphenyl)-1-(naphthalen-2-yl)-indole-2-carboxylic acid (37 mg, 67 nmol) in CH₂Cl₂ was added a stock solution of MeONa (3.37 M, 19.9 µL). The mixture was stirred at room temperature for 20 min. The precipitate was collected by filtration and dried *in vacuo* to afford the title compound (25 mg, 65%).
200 MHz ¹H-NMR (DMSO-d₆, ppm) δ 8.12-7.95 (4H, m), 7.76-7.65 (4H, m), 7.62-7.61 (6H, m), 7.00-6.87 (4H, m), 4.63 (1H, septet, J= 6.0 Hz), 4.57 (1H, septet, J= 6.0 Hz), 1.31 (6H, d, J=6.0 Hz), 1.25 (6H, d, J=6.0 Hz).

### Example 10

### 1,3,6-Tris(4-isopropoxyphenyl)indole-2-carboxylic acid

The title compound was prepared in accordance with Example 1, using 6-bromoindole-2-carboxylic acid ethyl ester, and 4-isopropoxyphenylboronic.

### Example 10A

### Sodium 1,3,6-tris(4-isopropoxyphenyl)-indole-2-carboxylate

The title compound was prepared in accordance with Example 9A from 1,3,6-tris(4-isopropoxyphenyl)indole-2-carboxylic acid (see Example 10). 200 MHz ¹H-NMR (DMSO-d₆, ppm) δ 7.71-5.59 (3H, m), 7.53-7.39 (4H, m), 7.32-7.27 (1H, m), 7.23-7.22 (1H, m), 7.06-6.89 (6H, m), 4.73-4.54 (3H, m), 1.35-1.23 (18H, m).

### Example 11

### 1-(6-Isopropoxypyrid-3-yl)-3-(pyrid-3-yl)-5-(4-(trifluoromethyl)phenyl)-indole-2-carboxylic acid

### (a) 3-Iodo-5-(4-(trifluoromethyl)phenyl)indole-2-carboxylic acid ethyl ester

The sub-title compound was prepared in accordance with Example 1(a) and I (b), using 5-bromoindole-2-carboxylic acid ethyl ester, 4-(trifluoromethyl)phenylboronic acid, NaI and *N*-chlorosuccinimide.

### (b) 3-(Pyrid-3-yl)-5-(4-(trifluoromethyl)phenyl)indole-2-carboxylic acid ethyl ester

Pd(PCy₃)₂ (0.055 mmol, 5.5 mL, 5 mol%) in dioxane was added to a stirred mixture of 3-iodo-5-(4-(trifluoromethyl)phenyl)indole-2-carboxylic acid ethyl ester (480 mg, 1.1 mmol; see step (a)), 3-(4,4,5,5-tetramethyl-1,3,2-dioxaborolan-2-yl)pyridine (248 mg, 1.21 mmol), Na₂CO₃ (aq. 2M, 2.75 µL, 5.5 mmol) and dioxane (13 mL) at room temperature. The mixture was stirred at 80°C for 16 h, allowed to cool, diluted with EtOAc and then washed with brine, dried over MgSO₄, concentrated and purified by chromatography to yield the sub-title compound (376 mg, 79%).

### (c) 5-Bromo-2-isopropoxypyridine

AgCO₃ (1.3 g, 5 mmol), toluene (15 mL) and 2-iodopropane (1.2 mL, 12 mmol) were added to 5-bromopyridin-2(1*H*)-one (1.74 g, 10 mmol). The mixture was stirred at 50 °C for 16 h, after which it was allowed to cool, then diluted with EtOAc and filtered through Celite^{®}. The filter cake was washed with EtOAc and the combined filtrates concentrated and purified by distillation to yield the sub-title compound (1.12 g, 52%).

### (d) 1-(6-Isopropoxypyrid-3-yl)-3-(pyrid-3-yl)-5-(4-(trifluoromethyl)-phgnyl)indole-2-carboxylic acid methyl ester

A mixture of CuI (7.6 mg, 40 nmol) and *N*,*N*'-dimethyl-1,2-diaminoethane (13 µL, 120 nmol) in toluene (0.5 mL) was added to a stirred mixture of 3-(pyrid-3-yl)-5-(4-(trifluoromethyl)phenyl)indole-2-carboxylic acid ethyl ester (164 mg, 400 nmol; see step (b)), 5-bromo-2-isopropoxypyridine (216 mg, 1 mmol; see step (c)), K₃PO₄ (196 mg, 800 nmol) and toluene (2 mL) at room temperature under argon. The mixture was stirred at 110°C for 5 h and at 140 °C for 16 h, after which it was allowed to cool and filtered through Celite^{®}. The filter cake was washed with EtOAc and the combined filtrates concentrated and purified by chromatography to yield the sub-title compound (57 mg, 29%).

### (e) 1-(6-Isopropoxypyrid-3-yl)-3-(pyrid-3-yl)-5-(4-(trifluoromethyl)-phenyl)indole-2-carboxylic acid

The title compound was prepared by hydrolysis of 1-(6-isopropoxypyrid-3-yl)-3-(pyrid-3-yl)-5-(4-(trifluoromethyl)phenyl)indole-2-carboxylic acid ethyl ester (see step (d)) in accordance with the procedure described in Example 1(e).
200 MHz ¹H-NMR (DMSO-d₆, ppm) δ 8.90-8.71 (1H, m), 8.65-8.43 (1H, m), 8.26 (1H, d, J=2.6 Hz), 8.10-7.99 (1H, m), 7.94-7.71 (6H, m), 7.66 (1H, d, J=8.8 Hz), 7.56-7.41 (1H, m), 7.23 (1H, d, J=8.8 Hz), 6.91 (1H, d, J=8.8 Hz), 5.30 (1H, septet, J=6.0 Hz), 1.34 (6H, d, J=6.0 Hz).

### Example 12

### 3-(6-Isopropoxypyrid-3-yl)-1-(4-(trifluoromethoxy)phenyl)-5-(4-(trifluoromethyl)phenyl)indole-2-carboxylic acid

### (a) 2-Isopropoxy-5-(4,4,5,5-tetramethyl-1,3,2-dioxaborolan-2-yl)pyridine

A mixture of 5-bromo-2-isopropoxypyridine (300 mg, 1.4 mmol; see Example 11(c)), KOAc (206 mg, 1.54 mmol) and bispinacolatodiborane (391 mg, 1.54 mmol) in dioxane (10 mL) was stirred under argon at 80 °C. A mixture of Pd₂(dba)₃ (46 mg, 50 nmol), PCy₃ (84 mg, 300 nmol) and anhydrous dioxane (5 mL) was added. The combined mixture was stirred at 80 °C for 16 h, after which it was allowed to cool and filtered through Celite^{®}. The filter cake was washed with EtOAc and the combined filtrates concentrated and purified by chromatography to yield the sub-title compound (216 mg, 59%).

### (b) 3-(6-Isopropoxypyrid-3-yl)-5-(4-(trifluorometyl)phenyl)indole-2-carboxylic acid ethyl ester

A mixture of Pd₂(dba)₃ (23 mg, 25 nmol) and PCy₃ (42 mg, 150 nmol) in anhydrous dioxane (5 mL) was stirred and then added to a mixture of 3-iodo-5-(4-(trifluoromethyl)phenyl)indole-2-carboxylic acid ethyl ester (326 mg, 0.71 mmol; see Example 11(a)), 2-isopropoxy-5-(4,4,5,5-tetramethyl-1,3,2-dioxaborolan-2-yl)pyridine (205 mg, 0.78 mmol; see step (a) above), Na₂CO₃ (aq. 2M, 1.17 mL, 2.34 mmol) and dioxane under argon at 80 °C. The mixture was stirred at 80 °C for 16 h, after which it was allowed to cool and extracted with EtOAc. The combined extracts were washed with brine, dried over MgSO₄, concentrated and purified by chromatography to yield the sub-title compound (219 mg, 66%).

### (c) 3-(6-Isopropoxypyrid-3-yl)-1-(4-(trifluoromethoxy)phenyl)-5-(4-(trifluoromethyl)phenyl)indole-2-carboxylic acid ethyl ester

The sub-title compound was prepared in accordance with the procedure described in Example 1(d) from 3-(6-isopropoxypyrid-3-yl)-5-(4-(trifluoromethyl)phenyl)indole-2-carboxylic acid ethyl ester (see step (b)) and 4-(trifluoromethoxy)phenylboronic acid.

### (d) 3-(6-Isopropoxypyrid-3-yl)-1-(4-(trifluoromethoxy)phenyl)-5-(4-(trifluoromethyl)phenyl)indole-2-carboxylic acid

The title compound was prepared by hydrolysis of 3-(6-isopropoxypyrid-3-yl)-1-(4-(trifluoromethoxy)phenyl)-5-(4-(trifluoromethyl)phenyl)indole-2-carboxylic acid ethyl ester (see step (c)) in accordance with the procedure described in Example 1 (e).
200 MHz ¹H-NMR (DMSO-d₆, ppm) δ 8.46-8.33 (1H, m), 7.98-7.71 (6H, m), 7.68-7.51 (5H, m), 7.26 (1H, d, J=8.8 Hz), 6.87-6.79 (1H, m), 5.30 (1H, septet, J=5.9 Hz), 1.32 (6H, d, J=5.9 Hz).

### Example 13

### 5-(4-tert-Butylphenyl)-3-(4-isopropoxyphenyl)-1-pyrid-3-yl-1H-indole-2-carboxylic acid

The title compound was prepared in accordance with Example 1(d) from 5-(4-*tert*-butylphenyl)-3-(4-*iso*-propoxyphenyl)-1*H*-indole-2-carboxylic acid ethyl ester (see Example 1(c)) and 3-(4,4,5,5-tetramethyl-[1,3,2]dioxaborolan-2-yl)-pyridine, followed by ester hydrolysis in accordance with the procedure described in Example 1(e).
200 MHz ¹H-NMR (DMSO-d₆, ppm) δ 8.72-8.52 (2H, m) 8.01-7.91 (1H, m) 7.72-7.38 (9H, m) 7.17 (1H, d, J=8.4 Hz) 7.07-6.95 (2H, m) 4.70 (1H, septet, J=6.1 Hz) 1.31 (6H, d, J=6.1 Hz) 1.28 (9H, s).

### Example 14

Title compounds of the examples were tested in the biological test described above and were found to exhibit 50% inhibition of mPGES-1 at a concentration of 10 µM or below. For example, for the following compounds of the examples, 50% inhibition was observed at:
Example 2: 350 nM
Example 5: 210 nM
Example 6: 70 nM
Example 11: 1800 nM
Example 13: 950 nM

## Claims

1. A compound of formula I, 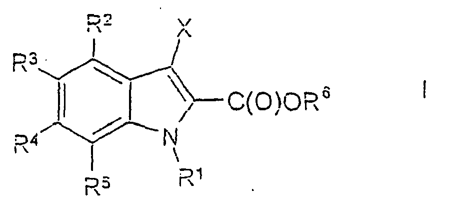 wherein
X and R¹ independently represent an aryl group or a heteroaryl group, both of which groups are optionally substituted by one or more substituents selected from A;
one of the groups R², R³, R⁴ and R⁵ represents an aryl group or a heteroaryl group (both of which are optionally substituted by one or more substituents selected from A) and:
a) the other groups are independently selected from hydrogen, G¹, an aryl group, a heteroaryl group (which latter two groups are optionally substituted by one or more substituents selected from A), C₁₋₈ alkyl and a heterocycloalkyl group (which latter two groups are optionally substituted by one or more substituents selected from G¹ and/or Z¹); and/or
b) any two other groups which are adjacent to each other are optionally linked to form, along with two atoms of the essential benzene ring in the compound of formula I, a 3- to 8-membered ring, optionally containing 1 to 3 heteroatoms and/or 1 to 3 double bonds, which ring is itself optionally substituted by one or more substituents selected from halo, -R⁶, -OR⁶ and =0;
*A* represents, on each occasion when mentioned above:
I) an aryl group or a heteroaryl group, both of which are optionally substituted by one or more substituents selected from B;
II) C₁₋₈ alkyl or a heterocycloalkyl group, both of which are optionally substituted by one or more substituents selected from G¹ and/or Z¹;
III) a G¹ group; or
IV) two A substituents may be linked together to form, along with at least two (e.g. adjacent) atoms of the aryl or heteroaryl group to which the two A substituents are attached, a further 3- to 5-membered ring, which, ring optionally contains 1 to 3 hetereoatoms and/or I to 2 double bonds, and which is optionally substituted by halo or C₁₋₈ alkyl, which latter group is optionally substituted by halo;
R⁶ represents, on each occasion when mentioned above:
I) hydrogen;
II) an aryl group or a heteroaryl group, both of which are optionally substituted by one or more substituents selected from B; or
III) C₁₋₈ alkyl or a heterocycloalkyl group, both of which are optionally substituted by one or more substituents selected from G¹ and/or Z¹;
G¹ represents, on each occasion when mentioned above, halo, cyano, -N₃, -NO₂, -ONO₂ or -A¹-R⁷;
wherein A¹ represents a single bond or a spacer group selected from
-C(O)A²-, -S(O)ₙA³-, -N(R⁸)A⁴- or -OA⁵-, in which:
A² and A³ independently represent a single bond, -O-, -N(R⁸)- or -C(O)-;
A⁴ and A⁵ independently represent a single bond, -C(O)-, -C(O)N(R⁸)-, -C(O)O-, -S(O)ₙ- or -S(O)ₙN(R⁸)-;
Z¹ represents, on each occasion when mentioned above, =O, =S, =NOR⁷, =NS(O)ₙN(R⁸)(R⁷), =NCN or =C(H)NO₂;
B represents, on each occasion when mentioned above:
I) an aryl group or a heteroaryl group, both of which are optionally substituted by one or more substituents selected from G², methylenedioxy, difluoromethylenedioxy and/or dimethylmethylenedioxy;
II) C₁₋₈ alkyl or a heterocycloalkyl group, both of which are optionally substituted by one or more substituents selected from G² and/or Z²;
III) a G² group; or
IV) methylenedioxy, difluoromethylenedioxy or dimethylmethylenedioxy;
G² represents, on each occasion when mentioned above, halo, cyano, -N₃, -NO₂, -ONO₂ or -A⁶-R⁹;
Wherein A⁶ represents a single bond or a spacer group selected from -C(O)A⁷-, -S(O)ₙA⁸-, -N(R¹⁰)A⁹- or -OA¹⁰-, in which:
A⁷ and A⁸ independently represent a single bond, -O-, -N(R¹⁰)- or -C(O)-;
A⁹ and A¹⁰ independently represent a single bond, -C(O)-, -C(O)N(R¹⁰)-, -C(O)O-, -S(O)ₙ-or-S(O)ₙN(R¹⁰)-;
Z² represents, on each occasion when mentioned above, =O, =S, =NOR⁹, =NS(O)ₙN(R¹⁰)(R⁹), =NCN or =C(H)NO₂;
R⁷, R⁸, R⁹ and R¹⁰ are independently selected from:
i) hydrogen;
ii) an aryl group or a heteroaryl group, both of which are optionally substituted by one or more substituents selected from G³, methylenedioxy, difluoromethylenedioxy and/or dimethylmethylenedioxy;
iii) C₁₋₈ alkyl or a heterocycloalkyl group, both of which are optionally substituted by G³ and/or Z³; or any pair of R⁷ and R⁸, or R⁹ and R¹⁰, may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from G³ and/or Z³;
G³ represents, on each occasion when mentioned above, halo, cyano, -N₃, -NO₂, -ONO₂ or -A¹¹-R¹¹;
wherein A¹¹ represents a single bond or a spacer group selected from -C(O)A¹²-, -S(O)ₙA¹³-, -N(R¹²)A¹⁴- or -OA¹⁵-, in which:
A¹² and A¹³ independently represent a single bond, -O-, -N(R¹²)- or -C(O)-;
A¹⁴ and A¹⁵ independently represent a single bond, -C(O)-, -C(O)N(R¹²)-, -C(O)O-, -S(O)ₙ- or -S(O)ₙN(R¹²)-;
Z³ represents, on each occasion when mentioned above, =O, =S, =NOR¹¹, =NS(O)ₙN(R¹²)(R¹¹), =NCN or =C(H)NO₂;
n represents, on each occasion when mentioned above, 1 or 2;
R¹¹ and R¹² are independently selected from:
i) hydrogen;
ii) C₁₋₆ alkyl or a heterocycloalkyl group, both of which groups are optionally substituted by one or more substituents selected from halo, C₁₋₄ alkyl, -N(R¹³)(R¹⁴), -O(R¹³) and =O; and
iii) an aryl or heteroaryl group, both of which are optionally substituted by one or more substituents selected from halo, C₁₋₄ alkyl, -N(R¹³)(R¹⁴) and -O(R¹³); or
any pair R¹¹ and R¹² may, for example when present on the same or on adjacent atoms, be linked together to form with those, or other relevant, atoms a further 3- to 8-membered ring, optionally containing 1 to 3 heteroatoms and/or 1 to 3 double bonds, which ring is optionally substituted by one or more substituents selected from halo, C₁₋₄ alkyl, -N(R¹³)(R¹⁴), -O(R¹³) and =O;
R¹³ and R¹⁴ are independently selected from hydrogen and C₁₋₄ alkyl, which latter group is optionally substituted by one or more halo groups;
or a pharmaceutically-acceptable salt thereof wherein each C_{1-q} alkyl group (where q is the upper limit of the range) may:
(a) be straight-chain or, when there is a sufficient number (i.e. a minimum of two) of carbon atoms, be branched-chain;
(b) when there is a sufficient number (i.e. a minimum of three) of carbon atoms, be cyclic (forming a C_{3-q} cycloalkyl group, including monocyclic or bicyclic C_{3-q} cycloalkyl groups, which cycloalkyl groups may further be bridged);
(c) when there is a sufficient number (i.e. a minimum of four) of carbon atoms, be part cyclic;
(d) be saturated or, when there is a sufficient number (i.e. a minimum of two) of carbon atoms, be unsaturated (forming a C_{3-q} cycloalkenyl, a C₈ cycloalkynyl, a C_{2-q} alkenyl or a C_{2-q} alkynyl group); and/or
(e) form a "spiro"-compound, in the case where a substituent on a cycloalkyl group is another cyclic compound, attached through a single atom,
.

2. A compound as claimed in Claim 1, wherein:
A² and A³ independently represent a single bond, -O- or -N(R⁸)-;
Z¹ represents, on each occasion when mentioned above, =O, =NOR⁷, =NS(O)ₙN(R⁸)(R⁷), =NCN or =C(H)NO₂;
A⁷ and A⁸ independently represent a single bond, -O- or -N(R¹⁰)-;
Z² represents, on each occasion when mentioned above, =O, =NOR⁹, =NS(O)ₙN(R¹⁰)(R⁹), =NCN or =C(H)NO₂;
A¹² and A¹³ independently represent a single bond, -O- or -N(R¹²)-; and/or Z³ represents, on each occasion when mentioned above, =O, =NOR¹¹, =NS(O)ₙN(R¹²)(R¹¹) , =NCN or =C(H)NO₂.

3. A compound as claimed in Claim 1 or Claim 2, wherein n represents 2.

4. A compound as claimed in any one of the preceding claims, wherein A represents G¹.

5. A compound as claimed in any one of the preceding claims, wherein G¹ represents halo, cyano, -NO₂ or -A¹-R⁷.

6. A compound as claimed in Claim 5, wherein G¹ represents -NO₂ or -A¹-R⁷.

7. A compound as claimed in any one of the preceding claims, wherein A¹ represents a single bond, -N(R⁸)A⁴- or -OA⁵-.

8. A compound as claimed in any one of the preceding claims, wherein A⁴ and A⁵ independently represent a single bond.

9. A compound as claimed in any one of the preceding claims, wherein R⁷ and R⁸ are independently selected from hydrogen and C₁₋₆ alkyl, which latter group is optionally substituted by one or more substituents selected from G³.

10. A compound as claimed in any one of the preceding claims, wherein G³ represents halo.

11. A compound as claimed in any one of the preceding claims, wherein R¹ represents an optionally substituted phenyl, naphthyl or pyridyl group.

12. A compound as claimed in any one of the preceding claims, wherein X represents an optionally substituted phenyl or pyridyl group.

13. A compound as claimed in any one of the preceding claims, wherein R³ and R⁴ independently represent G¹, hydrogen or an optionally substituted phenyl or pyridyl group.

14. A compound as claimed in Claim 13, wherein R³ and R⁴ independently represent hydrogen or an optionally substituted phenyl or pyridyl group.

15. A compound as claimed in any one of the preceding claims, wherein at least one of R³ and R⁴ represents optionally substituted phenyl or pyridyl, and up to one other represents G¹ or hydrogen.

16. A compound as claimed in any one of Claims 13 to 15, wherein when R³ or R⁴ represents an optionally substituted phenyl or pyridyl group, then the other substituents on the essential benzene ring of the indole of formula I, as defined in Claim 1, (i.e. R², R⁵ and R³ or R⁴ (as appropriate)) independently represent G¹ or hydrogen.

17. A compound as claimed in Claim 16, wherein the other substituents represent hydrogen.

18. A compound as claimed in any one of Claims 11 to 17, wherein the optional substituents are selected from cyano, heterocycloalkyl, halo, -NO₂, C₁₋₆ alkyl (which alkyl group may be linear or branched, cyclic, part-cyclic, unsaturated and/or optionally substituted with one or more halo group), -OR¹⁵ and -N(R¹⁵)R¹⁶, wherein R¹⁵ and R¹⁶ independently represent, H or C₁₋₆ alkyl (which alkyl group is optionally substituted by one or more halo groups)

19. A compound as claimed in Claim 18, wherein the optional substituents are selected from halo, -NO₂, C₁₋₆ alkyl (which alkyl group may be linear or branched, cyclic, part-cyclic, unsaturated and/or optionally substituted with one or more halo group), -OR¹⁵ and -N(R¹⁵)R¹⁶, wherein
R¹⁵ and R¹⁶ independently represent, H or C₁₋₆ alkyl (which alkyl group is optionally substituted by one or more halo groups).

20. A compound as claimed in any one of the preceding claims, wherein R⁶ represents hydrogen.

21. A compound as defined in any one of Claims 1 to 20, or a pharmaceutically-acceptable salt thereof, for use as a pharmaceutical.

22. A pharmaceutical formulation including a compound as defined in any one of Claims 1 to 20, or a pharmaceutically-acceptable salt thereof, in admixture with a pharmaceutically acceptable adjuvant, diluent or carrier.

23. A compound as defined in any one of Claims 1 to 20, or a pharmaceutically-acceptable salt thereof, for the treatment of a disease in which inhibition of the activity of microsomal prostaglandin E synthase-1, leukotriene C₄ synthase and/or 5-lipoxygenase-activaing protein is desired and/or required.

24. A compound as claimed in Claim 23, wherein inhibition of the activity of microsomal prostaglandin E synthase-1 is desired and/or required.

25. The use of a compound as defined in any one of Claims 1 to 20, or a pharmaceutically-acceptable salt thereof, for the manufacture of a medicament for the treatment of a disease in which inhibition of the activity of microsomal prostaglandin E synthase-1, leukotriene C₄ synthase and/or 5-lipoxygenase-activaing protein is desired and/or required.

26. A use as claimed in Claim 25, wherein the disease is inflammation.

27. A use as claimed in Claim 26 wherein the disease is inflammatory bowel disease, irritable bowel syndrome, migraine, headache, low back pain, fibromyalgia, a myofascial disorder, a viral infection, a bacterial infection, a fungal infection, dysmenorrhea, a bum, a surgical or dental procedure, a malignancy, atherosclerosis, gout, arthritis, osteoarthritis, juvenile arthritis, rheumatoid arthritis, fever, ankylosing spondylitis, systemic lupus esythematosus, vasculitis, pancreatitis, nephritis, bursitis, conjunctivitis, iritis, scleritis, uveitis, wound healing, dermatitis, eczema, psoriasis, stroke, diabetes mellitus, a neurodegenerative disorder, an autoimmune disease, osteoporosis, asthma, chronic obstructive pulmonary disease, pulmonary fibrosis, an allergic disorder, rhinitis, an ulcer, coronary heart disease, sarcoidosis, inflammatory pain, hyperprostaglandin E syndrome, classic Bartter syndrome, Hodgkin's disease, persistent ductus, any other disease with an inflammatory component, Paget's disease or a periodontal disease.

28. A combination product comprising:
(A) a compound as defined in any one of Claims 1 to 20, or a pharmaceutically-acceptable salt thereof; and
(B) another therapeutic agent that is useful in the treatment of inflammation,
wherein each of components (A) and (B) is formulated in admixture with a pharmaceutically-acceptable adjuvant, diluent or carrier.

29. A combination product as claimed in Claim 28 which comprises a pharmaceutical formulation including a compound as defined in any one of Claims 1 to 20, or a pharmaceutically-acceptable salt thereof, another therapeutic agent that is useful in the treatment of inflammation, and a pharmaceutically-acceptable adjuvant, diluent or carrier.

30. A combination product as claimed in Claim 28 which comprises a kit of parts comprising components:
(a) a pharmaceutical formulation including a compound as defined in any one of Claims 1 to 20, or a phaimaceutically-acceptable salt thereof, in admixture with a pharmaceutically-acceptable adjuvant, diluent or carrier; and
(b) a pharmaceutical formulation including another therapeutic agent that is useful in the treatment of inflammation in admixture with a pharmaceutically-acceptable adjuvant, diluent or carrier,
which components (a) and (b) are each provided in a form that is suitable for administration in conjunction with the other.

## Patentansprüche

1. Eine Verbindung gemäß Formel I, 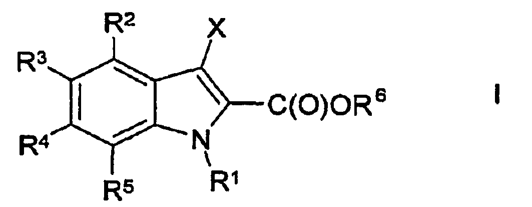 in welcher
X und R¹ voneinander unabhängig eine Aryl-Gruppe oder eine Heteroaryl-Gruppe bedeuten, wobei beide Gruppen optional durch einen oder mehrere Substituenten substituiert sind, die aus A ausgewählt sind,
eine der Gruppen R2, R3, R4 und R5 eine Aryl-Gruppe oder eine Heteroaryl-Gruppe bedeutet (, von denen beide optional durch einen oder mehrere aus A ausgewählten Substituenten substituiert sind), und:
a) die anderen Gruppen unabhängig voneinander ausgewählt sind aus Wasserstoff, G¹, einer Aryl-Gruppe, einer Heteroaryl-Gruppe (wobei die beiden letztgenannten Gruppen optional durch einen oder mehrere aus A ausgewählte Substituenten substituiert sind), C₁₋₈-Alkyl- oder einer Heterocycloalkyl-Gruppe (wobei die beiden letztgenannten Gruppen optional durch einen oder mehrere Substituenten substituiert sind, die aus G¹ und/oder Z¹ ausgewählt sind); und/oder
b) irgend zwei andere Gruppen, die einander benachbart sind, optional verbunden sind, um zusammen mit zwei Atomen des essentiellen Benzen-Rings in der Verbindung gemäß Formel I einen 3- bis 8-gliedrigen Ring zu bilden, der optional 1 bis 3 Heteroatome und/oder 1 bis 3 Doppelbindungen enthält, wobei der Ring selbst optional durch einen oder mehrere Substituenten substituiert ist, die aus Halo, -R⁶, - OR⁶ und =O ausgewählt sind;
A überall dort, wo es oben erwähnt wird:
I) eine Aryl-Gruppe oder eine Heteroaryl-Gruppe, von denen beide optional durch einen oder mehrere aus B ausgewählte Substituenten substituiert sind;
II) C₁₋₈-Alkyl oder eine Heterocycloalkyl-Gruppe, von denen beide optional durch einen oder mehrere Substituenten substituiert sind, die aus G¹ und/oder Z¹ ausgewählt sind;
III) oder eine G¹-Gruppe bedeutet; oder
IV) zwei A-Substituenten miteinander verknüpft sein können, um gemeinsam mit wenigstens zwei (z.B. benachbarten) Atomen der Aryl- oder Heteroaryl-Gruppe, an welche die beiden A-Substituenten angehängt sind, einen weiteren 3- bis 5-gliedrigen Ring zu bilden, wobei dieser Ring optional 1 bis 3 Heteroatome und/oder 1 bis 2 Doppelbindungen enthält, und optional durch Halo oder C₁₋₈-Alkyl substituiert ist,
wobei die letztgenannte Gruppe optional durch Halo substituiert ist;
R⁶ überall dort, wo es oben erwähnt wird
I) Wasserstoff;
II) eine Aryl-Gruppe oder eine Heteroaryl-Gruppe, von denen beide optional durch einen oder mehrere Substituenten substituiert sind, die aus B ausgewählt sind; oder;
III) C₁₋₈-Alkyl oder eine Heterocycloalkyl-Gruppe bedeutet, von denen beide optional durch einen oder mehrere Substituenten substituiert sind, die aus G¹ und/oder Z¹ ausgewählt sind, oder
G¹ überall dort, wo es oben erwähnt wird, Halo, Cyano, -N₃, -NO₂, -ONO₂ oder -A¹-R⁷ bedeutet,
wobei A¹ eine Einzelbindung oder eine Abstandsgruppe bedeutet, die aus -C(O)A²-, -S(O)ₙA³-, -N(R⁸)A⁴- oder -OA⁵- ausgewählt ist, wobei:
A² und A³ voneinander unabhängig eine Einzelbindung, -O-, -N(R⁸)- oder -C(O)- bedeuten und
A⁴ und A⁵ voneinander unabhängig eine Einzelbindung, -C(O)-, -C(O)N(R⁸)-, - C(O)O-, -S(O)ₙ- oder -S(O)ₙN(R⁸)- bedeuten;
Z¹ überall dort, wo es oben erwähnt wird, =O, =S, =NOR⁷, =NS(O)ₙN(R⁸)(R⁷), =NCN oder =C(H)NO₂ bedeutet;
B überall dort, wo es oben erwähnt wird:
I) eine Aryl-Gruppe oder eine Heteroaryl-Gruppe, von denen beide optional durch einen oder mehrere Substituenten substituiert sind, die aus G², Methylendioxy, Difluoromethylendioxy und/oder Dimethylmethylendioxy ausgewählt sind;
II) eine C₁₋₈-Alkyl- oder eine Heterocycloalkyl-Gruppe, von denen beide optional durch einen oder mehrere Substituenten substituiert sind, die aus G² und/oder Z² ausgewählt sind;
III) eine G²-Gruppe; oder
IV) Methylendioxy, Difluoromethylendioxy oder Dimethylmethylendioxy bedeutet;
G² überall dort, wo es oben erwähnt wird, Halo, Cyano, -N₃,-NO₂, -ONO₂ oder -A⁶-R⁹ bedeutet,
wobei A⁶ eine Einzelbindung oder eine Abstandsgruppe bedeutet, die aus -C(O)A⁷-, -S(O)ₙA⁸-, -N(R¹⁰)A⁹- oder -OA¹⁰- ausgewählt ist, wobei:
A⁷ und A⁸ unabhängig voneinander eine Einzelbindung, -O-, -N(R¹⁰)- oder -C(O)-bedeuten; und
A⁹ und A¹⁰ unabhängig voneinander eine Einzelbindung, -C(O)-, -C(O)N(R¹⁰)-, -C(O)O-, -S(O)ₙ- oder -S(O)ₙN(R¹⁰)- bedeuten;
Z² überall dort, wo es oben verwendet wird, =O, =S, =NOR⁹, =NS(O)ₙN(R¹⁰)(R⁹), =NCN oder =C(H)NO₂ bedeutet;
R⁷, R⁸, R⁹ und R¹⁰ voneinander unabhängig ausgewählt sind aus:
i) Wasserstoff;
ii) einer Aryl-Gruppe oder einer Heteroaryl-Gruppe, von denen beide optional durch einen oder mehrere Substituenten substituiert sind, die aus G³, Methylendioxy, Difluoromethylendioxy und/oder Dimethylmethylendioxy ausgewählt sind;
iii) einer C₁₋₈-Alkyl- oder eine Heterocycloalkyl-Gruppe, von denen beide optional durch G³ und/oder Z³ substituiert sind; oder wobei
irgendein Paar von R⁷ und R⁸, oder R⁹ und R¹⁰, beispielsweise dann, wenn sie an dem gleichen oder an benachbarten Atomen vorhanden sind, miteinander verknüpft sein können, um mit diesen oder anderen relevanten Atomen einen weiteren 3- bis 8-gliedrigen Ring zu bilden, der optional 1 bis 3 Heteroatome und/oder 1 bis 3 Doppelbindungen enthält, wobei dieser Ring optional durch einen oder mehrere Substituenten substituiert ist, die aus G³ und/oder Z³ ausgewählt sind;
G³ überall dort, wo es oben erwähnt wird Halo, Cyano, -N₃, -NO₂, -ONO₂ oder -A¹¹-R¹¹ bedeutet;
wobei A¹¹ eine Einzelbindung oder eine Abstands-Gruppe bedeutet, die aus -C(O)A¹²-, -S(O)ₙA¹³-, -N(R¹²)A¹⁴- oder -OA¹⁵- ausgewählt ist, wobei:
A¹² und A¹³ unabhängig voneinander eine Einzelbindung, -O-, -N(R¹²)- oder - C(O)- bedeuten; und
A¹⁴ und A¹⁵ unabhängig voneinander eine Einzelbindung, -C(O)-, - C(O)N(R¹²)-, -C(O)O-, -S(O)ₙ- oder -S(O)ₙN(R¹²)- bedeuten;
Z³ überall dort, wo es oben erwähnt wird, =O, =S, =NOR¹¹, =NS(O)ₙN(R¹²)(R¹¹), =NCN oder =C(H)NO₂ bedeutet;
n überall dort, wo es oben erwähnt wird, entweder 1 oder 2 bedeutet;
R¹¹ und R¹² unabhängig voneinander ausgewählt sind aus:
i) Wasserstoff;
ii) einer C₁₋₆-Alkyl- oder einer Heterocycloalkyl-Gruppe, von denen beide Gruppen optional durch einen oder mehrere Substituenten substituiert sind, die aus Halo, C₁₋₄-Alkyl, -N(R¹³)(R¹⁴), -O(R¹³) und =O ausgewählt sind; und
iii) einer Aryl- oder einer Heteroaryl-Gruppe, von denen beide optional durch einen oder mehrere Substituenten substituiert sind, die aus Halo, C₁₋₄-Alkyl, -N(R¹³)(R¹⁴) und -O (R¹³) ausgewählt sind; oder
irgend ein Paar R¹¹ und R¹² dann, wenn es beispielsweise an den gleichen oder an benachbarten Atomen vorhanden ist, verbunden sein kann, um mit diesen oder anderen relevanten Atomen einen weiteren 3- bis 8-gliedrigen Ring zu bilden, der optional 1 bis 3 Heteroatome und/oder 1 bis 3 Doppelbindungen enthält, wobei dieser Ring optional durch einen oder mehrere Substituenten substituiert ist, die aus Halo, C₁₋₄-Alkyl, -N(R¹³)(R¹⁴), -O(R¹³) und =O ausgewählt sind; und wobei
R¹³ und R¹⁴ unabhängig voneinander ausgewählt sind aus Wasserstoff und C₁₋₄-Alkyl, wobei die letztgenannte Gruppe optional durch eine oder mehrere Halo-Gruppen substituiert ist,
wobei jede C_{1-q}-Alkylgruppe (wobei q die obere Bereichsgrenze ist) folgendes sein kann:
(a) eine gerade Kette oder, wenn eine ausreichende Anzahl von Kohlenstoffatomen (d.h. wenigstens 2) vorhanden ist, eine verzweigte Kette,
(b) zyklisch, wenn eine ausreichende Anzahl von Kohlenstoffatomen (d.h. wenigstens drei) vorhanden ist, (wobei eine C_{3-q}-Cycloalkyl-Gruppe gebildet wird; dies kann auch eine mono- oder bizyklische C_{3-q}-Cycloalkyl-Gruppe sein, wobei diese Cycloalkyl-Gruppen weiter überbrückt sein können),
(c) dann, wenn eine ausreichende Anzahl von Kohlenstoffatomen (d.h. wenigstens vier) vorhanden ist, teilzyklisch,
(d) gesättigt, oder dann, wenn eine ausreichende Anzahl von Kohlenstoffatomen (d.h. wenigstens zwei) vorhanden ist, ungesättigt (wobei eine C_{3-q}-Cycloalkenyl-, eine C₈-Cycloalkynyl-, eine C_{2-q}-Alkenyl- oder eine C_{2-q}-Alkynyl-Gruppe gebildet wird, uns/oder
(e) eine "Spiro"-Verbindung in dem Fall gebildet werden kann, in welchem ein Substituent an einer Cycloalkyl-Gruppe eine weitere, durch ein einzelnes Atom angehängte zyklische Verbindung ist oder ein pharmazeutisch akzeptables Salz hiervon,

2. Verbindung nach Anspruch 1, bei der
A² und A³ voneinander unabhängig eine Einzelbindung, -O- oder -N(R⁸)- bedeuten;
Z¹ überall dort, wo es oben erwähnt wird, =O, =NOR⁷, =NS(O)ₙN(R⁸)(R⁷), =NCN oder =C(H)NO₂ bedeutet;
A⁷ und A⁸ voneinander unabhängig eine Einzelbindung, -O- oder -N(R¹⁰)- bedeuten;
Z² überall dort, wo es oben erwähnt wird, =O, =NOR⁹, =NS(O)ₙN(R¹⁰)(R⁹), =NCN oder =C(H)NO₂ bedeutet;
A¹² und A¹³ unabhängig voneinander eine Einzelbindung, -O- oder -N(R¹²)- bedeuten; und/oder
Z³ überall dort, wo es oben erwähnt wird, =O, =NOR¹¹, =NS(O)ₙN(R¹²)(R¹¹), =NCN oder =C(H)NO₂ bedeutet.

3. Verbindung nach Anspruch 1 oder 2, bei der n gleich 2 ist.

4. Verbindung nach einem der vorhergehenden Ansprüche, bei der A für G¹ steht.

5. Verbindung nach einem der vorhergehenden Ansprüche, bei der G¹ für Halo, Cyano, -NO₂ oder -A¹-R⁷ steht.

6. Verbindung nach Anspruch 5, bei der G¹ für -NO₂ oder -A¹-R⁷ steht.

7. Verbindung nach einem der vorhergehenden Ansprüche, bei der A¹ eine Einzelbindung, -N(R⁸)A⁴- oder -OA⁵- bedeutet.

8. Verbindung nach einem der vorhergehenden Ansprüche, bei der A⁴ und A⁵ voneinander unabhängig eine Einzelbindung bedeuten.

9. Verbindung nach einem der vorhergehenden Ansprüche, bei der R⁷ und R⁸ voneinander unabhängig aus Wasserstoff und C₁₋₆-Alkyl ausgewählt sind, wobei die zuletzt genannte Gruppe optional durch einen oder mehrere aus G³ ausgewählte Substituenten substituiert ist.

10. Verbindung nach einem der vorhergehenden Ansprüche, bei der G³ für Halo steht.

11. Verbindung nach einem der vorhergehenden Ansprüche, bei der R¹ für eine optional substituierte Phenyl-, Naphthyl- oder Pyridyl-Gruppe steht.

12. Verbindung nach einem der vorhergehenden Ansprüche, bei der X für eine optional substituierte Phenyl- oder Pyridyl-Gruppe steht.

13. Verbindung nach einem der vorhergehenden Ansprüche, bei der R³ und R⁴ voneinander unabhängig für G¹, Wasserstoff oder eine optional substituierte Phenyl-oder Pyridyl-Gruppe stehen.

14. Verbindung nach Anspruch 13, bei der R³ und R⁴ voneinander unabhängig für Wasserstoff oder eine optional substituierte Phenyl- oder Pyridyl-Gruppe stehen.

15. Verbindung nach einem der vorhergehenden Ansprüche, bei R³ und/oder R⁴ für eine optional substituierte Phenyl- oder Pyridyl-Gruppe stehen und bis zu einer anderen G¹ oder Wasserstoff bedeuten.

16. Verbindung nach einem der Ansprüche 13 bis 15, bei der dann, wenn R³ oder R⁴ eine optional substituierte Phenyl- oder Pyridyl-Gruppe bedeutet, die anderen Substituenten an dem essentiellen Benzen-Ring des Indols aus Formel I, wie in Anspruch 1 definiert (d.h. R², R⁵ und R³ oder R⁴ (gegebenenfalls)) voneinander unabhängig G¹ oder Wasserstoff bedeuten.

17. Verbindung nach Anspruch 16, bei der die anderen Substituenten für Wasserstoff stehen.

18. Verbindung nach einem der Ansprüche 11 bis 17, bei der die optionalen Substituenten aus ausgewählt sind aus: Herterocycloalkyl, Halo, -NO₂, C₁₋₆-Alkyl (wobei diese Alkyl-Gruppe linear oder verzweigt, zyklisch, teil-zyklisch, ungesättigt und/oder optional mit einer oder mehreren Halo-Gruppen substituiert sein kann), -OR¹⁵ und - N(R¹⁵)R¹⁶, wobei R¹⁵ und R¹⁶ voneinander unabhängig für H oder C₁₋₆-Alkyl stehen (wobei diese Alkyl-Gruppe optional durch eine oder mehrere Halo-Gruppen substituiert ist).

19. Verbindung nach Anspruch 18, bei der die optionalen Substituenten ausgewählt sind aus: Halo, -NO₂, C₁₋₆-Alkyl (wobei diese Alkyl-Gruppe linear oder verzweigt, zyklisch, teil-zyklisch, ungesättigt und/oder optional mit einer oder mehreren Halo-Gruppen substituiert sein kann), -OR¹⁵ und -N(R¹⁵)R¹⁶, wobei R¹⁵ und R¹⁶ voneinander unabhängig für H oder C₁₋₆-Alkyl stehen (wobei diese Alkyl-Gruppe optional durch eine oder mehrere Halo-Gruppen substituiert ist).

20. Verbindung nach einem der vorhergehenden Ansprüche, bei der R⁶ für Wasserstoff steht.

21. Verbindung nach einem der Ansprüche 1 bis 20, oder ein pharmazeutisch akzeptables Salz hiervon zur Verwendung als Pharmazeutikum.

22. Pharmazeutische Zubereitung, die eine Verbindung umfasst, wie sie in einem der Ansprüche 1 bis 20 definiert ist, oder ein pharmazeutisch akzeptables Salz hiervon in Mischung mit einem pharmazeutisch akzeptablen Zusatzstoff, Verdünner oder Träger.

23. Verbindung nach einem der Ansprüche 1 bis 20, oder pharmazeutisch akzeptables Salz hiervon zur Behandlung einer Erkrankung, bei der die Hemmung der Aktivität von mikrosomaler Prostaglandin-E-Synthase-1, von Leukotrien-C₄-Synthase und/oder einem 5-Lipoxygenase aktivierendem Protein erwünscht und/oder erforderlich ist.

24. Verbindung nach Anspruch 23, bei der die Hemmung der Aktivität von mikrosomaler Prostaglandin-E-Synthase-1 erwünscht und/oder erforderlich ist.

25. Verwendung einer Verbindung nach einem der Ansprüche 1 bis 20, oder eines pharmazeutisch akzeptablen Salzes hiervon für die Herstellung eines Medikaments zur Behandlung einer Erkrankung, bei der die Hemmung der Aktivität von mikrosomaler Prostaglandin-E-Synthase-1, von Leukotrien-C₄-Synthase und/oder einem 5-Lipoxygenase aktivierendem Protein erwünscht und/oder erforderlich ist.

26. Verwendung nach Anspruch 25, wobei die Erkrankung eine Entzündung ist.

27. Verwendung nach Anspruch 26, wobei die Erkrankung eine entzündliche Darm-Erkrankung, das Reizdarm-Syndrom, Migräne, Kopfschmerzen, Schmerzen im unteren Rücken, Fibromyalgie, eine myofasciale Erkrankung, eine Virusinfektion, eine bakterielle Infektion, eine Pilzinfektion, Dysmenorrhö, eine Verbrennung, eine chirurgische Behandlung oder Zahnbehandlung, eine bösartige Erkrankung, Atherosklerose, Gicht, Arthritis, Osteoarthritis, jugendliche Arthritis, rheumatoide Arthritis, Fieber, Spondylitis ankylosans, systemischer Lupus erythematosus, eine Gefäßentzündung, eine Entzündung der Bauchspeicheldrüse, eine Nierenentzündung, eine Schleimbeutelentzündung, eine Bindehautentzündung, eine Regenbogenhautentzündung, eine Scleritis, eine Augenhaut-Entzündung, eine Wundheilungs-Dermatitis, ein Ekzem, eine Psoriasis, ein Schlaganfall, Diabetes mellitus, eine neurodegenerative Erkrankung, eine Autoimmun-Erkrankung, Osteoporose, Asthma, eine chronische obstruktive Pulmonar-Erkrankung, Pulmonar-Fibrose, eine allergische Erkrankung, eine Nasenschleimhaut-Entzündung, ein Geschwür, eine Herzkranz-Erkrankung, Sarkoidose, Entzündungsschmerzen, das Hyperprostaglandin-E-Syndrom, das klassische Bartter-Syndrom, die Hodgkins Erkrankung, ein persistierender Ductus, irgendeine andere Erkrankung mit einer entzündlichen Komponente, Paget's Erkrankung oder eine periodontale Erkrankung ist.

28. Kombinationsprodukt, das Folgendes umfasst:
(A) eine Verbindung nach einem der Ansprüche 1 bis 20, oder ein pharmazeutisch akzeptables Salz hiervon; und
(B) einen weiteren therapeutischen Wirkstoff, der bei der Behandlung von Entzündungen nützlich ist,
wobei jede der Komponenten (A) und (B) in Mischung mit einem pharmazeutisch akzeptablen Zusatzstoff, Verdünner oder Träger zubereitet ist.

29. Kombinationsprodukt nach Anspruch 28, das eine pharmazeutische Zubereitung umfasst, die eine Verbindung nach einem der Ansprüche 1 bis 20, oder ein pharmazeutisch akzeptables Salz hiervon, einen weiteren therapeutischen Wirkstoff, der bei der Behandlung von Entzündungen nützlich ist, und einen pharmazeutisch akzeptablen Zusatzstoff, Verdünner oder Träger umfasst.

30. Kombinationsprodukt nach Anspruch 28, das einen Teilesatz umfasst, der folgende Komponenten aufweist:
(a) eine pharmazeutische Zubereitung, die eine Verbindung nach einem der Ansprüche 1 bis 20, oder ein pharmazeutisch akzeptables Salz hiervon in Mischung mit einem pharmazeutisch akzeptablen Zusatzstoff, Verdünner oder Träger umfasst, und
(b) eine pharmazeutische Zubereitung, die einen weiteren therapeutischen Wirkstoff umfasst, der bei der Behandlung von Entzündungen nützlich ist, in Mischung mit einem pharmazeutisch akzeptablen Zusatzstoff, Verdünner oder Träger,
wobei die Komponenten (a) und (b) jeweils in einer Form vorgesehen sind, die für eine Verabreichung in Verbindung mit der anderen geeignet ist.

## Revendications

1. Composé de formule I, 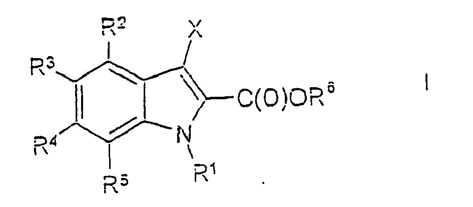 dans laquelle
X et R¹ représentent indépendamment un groupe aryle ou un groupe hétéroaryle, qui sont tous deux éventuellement substitués par un ou plusieurs substituants choisis parmi A ;
l'un des groupes R², R³, R⁴ et R⁵ représente un groupe aryle ou un groupe hétéroaryle (qui sont tous deux éventuellement substitués par un ou plusieurs substituants choisis parmi A) et :
a) les autres groupes sont choisis indépendamment parmi un hydrogène, G¹, un groupe aryle, un groupe hétéroaryle (ces deux derniers groupes étant éventuellement substitués par un ou plusieurs substituants choisis parmi A), un groupe alkyle en C₁-C₈ et un groupe hétérocycloalkyle (ces deux derniers groupes étant éventuellement substitués par un ou plusieurs substituants choisis parmi G¹ et/ou Z¹) ; et/ou
b) deux autres groupes quelconques qui sont adjacents l'un à l'autre sont éventuellement liés pour former, avec les deux atomes du noyau benzène essentiel dans le composé de formule I, un cycle de 3 à 8 chaînons, contenant éventuellement 1 à 3 hétéroatomes et/ou 1 à 3 doubles liaisons, cycle qui est lui-même éventuellement substitué par un ou plusieurs substituants choisis parmi halogéno, -R⁶, -OR⁶ et =O ;
A représente, dans chaque cas cité ci-dessus :
I) un groupe aryle ou un groupe hétéroaryle, qui sont tous deux éventuellement substitués par un ou plusieurs substituants choisis parmi B ;
II) un groupe alkyle en C₁-C₈ ou un groupe hétérocycloalkyle, qui sont tous deux éventuellement substitués par un ou plusieurs substituants choisis parmi G¹ et/ou Z¹ ;
III) un groupe G¹ ; ou
IV) deux substituants A peuvent être liés l'un à l'autre pour former, avec au moins deux atomes (par exemple adjacents) du groupe aryle ou hétéroaryle auquel les deux substituants A sont fixés, un autre cycle de 3 à 5 chaînons, cycle qui contient éventuellement 1 à 3 hétéroatomes et/ou 1 à 2 doubles liaisons, et qui est éventuellement substitué par halogéno ou alkyle en C₁-C₈, ce dernier groupe étant éventuellement substitué par un groupe halogéno ;
R⁶ représente, dans chaque cas cité ci-dessus :
I) un hydrogène ;
II) un groupe aryle ou un groupe hétéroaryle, qui sont tous deux éventuellement substitués par un ou plusieurs substituants choisis parmi B ; ou
III) un groupe alkyle en C₁-C₈ ou un groupe hétérocycloalkyle, qui sont tous deux éventuellement substitués par un ou plusieurs substituants choisis parmi G¹ et/ou Z¹ ;
G¹ représente, dans chaque cas cité ci-dessus, halogéno, cyano, -N₃, -NO₂, -ONO₂ ou -A¹-R⁷ ;
où A¹ représente une liaison simple ou un groupe espaceur choisi parmi -C(O)A²-, -S(O)ₙA³-, -N(R⁸)A⁴-ou -OA⁵-, où :
A² et A³ représentent indépendamment une liaison simple, -O-, -N(R⁸)- ou -C(O)- ;
A⁴ et A⁵ représentent indépendamment une liaison simple, -C(O)-, -C(O)N(R⁸)-, -C(O)O-, -S(O)ₙ-ou -S(O)ₙN(R⁸)- ;
Z¹ représente, dans chaque cas cité ci-dessus, =O, =S, =NOR⁷, =NS(O)ₙN(R⁸)(R⁷), =NCN ou =C(H)NO₂ ;
B représente, dans chaque cas cité ci-dessus :
I) un groupe aryle ou un groupe hétéroaryle, qui sont tous deux éventuellement substitués par un ou plusieurs substituants choisis parmi G², méthylènedioxy, difluorométhylènedioxy et/ou diméthylméthylènedioxy ;
II) un groupe alkyle en C₁-C₈ ou un groupe hétérocycloalkyle, qui sont tous deux éventuellement substitués par un ou plusieurs substituants choisis parmi G² et/ou Z² ;
III) un groupe G² ; ou
IV) un groupe méthylènedioxy, difluorométhylènedioxy ou diméthylméthylènedioxy ;
G² représente, dans chaque cas cité ci-dessus, halogéno, cyano, -N₃, -NO₂, -ONO₂ ou -A⁶-R⁹ ;
où A⁶ représente une liaison simple ou un groupe espaceur choisi parmi -C(O)A⁷-, -S(O)ₙA⁸-, -N(R¹⁰)A⁹-ou -OA¹⁰, où :
A⁷ et A⁸ représentent indépendamment une liaison simple, -O-, -N(R¹⁰)- ou -C(O)- ;
A⁹ et A¹⁰ représentent indépendamment une liaison simple, -C(O)-, -C(O)N(R¹⁰)-, -C(O)O-, -S(O)ₙ-ou -S(O)ₙN(R¹⁰)- ;
Z² représente, dans chaque cas cité ci-dessus, =O, =S, =NOR⁹, =NS(O)ₙN(R¹⁰)(R⁹), =NCN ou =C(H)NO₂ ;
R⁷, R⁸, R⁹ et R¹⁰ sont choisis indépendamment parmi :
i) un hydrogène ;
ii) un groupe aryle ou un groupe hétéroaryle, qui sont tous deux éventuellement substitués par un ou plusieurs substituants choisis parmi G³, méthylènedioxy, difluorométhylènedioxy et/ou diméthylméthylènedioxy ;
iii) un groupe alkyle en C₁-C₈ ou un groupe hétérocycloalkyle, qui sont tous deux éventuellement substitués par un ou plusieurs substituants choisis parmi G³ et/ou Z³ ;
une paire R⁷ et R⁸, ou R⁹ et R¹⁰, quelconque peut, par exemple, lorsqu'elle est présente sur le même atome ou sur des atomes adjacents, être liée l'un à l'autre pour former, avec ces atomes ou avec d'autres atomes en question, un autre cycle de 3 à 8 chaînons, contenant éventuellement 1 à 3 hétéroatomes et/ou 1 à 3 doubles liaisons, cycle qui est éventuellement substitué par un ou plusieurs substituants choisis parmi G³ et/ou Z³ ;
G³ représente, dans chaque cas cité ci-dessus, halogéno, cyano, -N₃, -NO₂, -ONO₂ ou -A¹¹-R¹¹ ;
où A¹¹ représente une liaison simple ou un groupe espaceur choisi parmi -C(O)A¹²-, -S(O)ₙA¹³-, -N(R¹²)A¹⁴ ou -OA¹⁵, où :
A¹² et A¹³ représentent indépendamment une liaison simple, -O-, -N(R¹²)- ou -C(O)- ;
A¹⁴ et A¹⁵ représentent indépendamment une liaison simple, -C(O)-, -C(O)N(R¹²)-, -C(O)O-, -S(O)ₙ-ou -S(O)ₙN(R¹²)- ;
Z³ représente, dans chaque cas cité ci-dessus, =O, =S, =NOR¹¹, =NS(O)ₙN(R¹²)(R¹¹), =NCN ou =C(H)NO₂ ;
n représente, dans chaque cas cité ci-dessus, 1 ou 2 ;
R¹¹ et R¹² sont choisis indépendamment parmi :
i) un hydrogène ;
ii) un groupe alkyle en C₁-C₆ ou un groupe hétérocycloalkyle, qui sont tous deux éventuellement substitués par un ou plusieurs substituants choisis parmi halogéno, alkyle en C₁-C₄, -N(R¹³)(R¹⁴), -O(R¹³) et =0 ; et
iii) un groupe aryle ou un groupe hétéroaryle, qui sont tous deux éventuellement substitués par un ou plusieurs substituants choisis parmi halogéno, alkyle en C₁-C₄, -N(R¹³)(R¹⁴) et -O(R¹³) ; ou
une paire R¹¹ et R¹² quelconque peut, par exemple, lorsqu'elle est présente sur le même atome ou sur des atomes adjacents, être liée l'un à l'autre pour former, avec ces atomes ou avec d'autres atomes en question, un autre cycle de 3 à 8 chaînons, contenant éventuellement 1 à 3 hétéroatomes et/ou 1 à 3 doubles liaisons, cycle qui est éventuellement substitué par un ou plusieurs substituants choisis parmi halogéno, alkyle en C₁-C₄, -N(R¹³)(R¹⁴), -O(R¹³) et =O ;
R¹³ et R¹⁴ sont choisis indépendamment parmi un hydrogène et un groupe alkyle en C₁-C₄, ce dernier groupe étant éventuellement substitué par un ou plusieurs groupes halogéno ;
ou un sel pharmaceutiquement acceptable de celui-ci, où chaque groupe alkyle en C₁-C_{q} (où q est la limite supérieure de la plage) peut être :
(a) à chaîne droite ou, lorsque le nombre d'atomes de carbone est suffisant (à savoir un minimum de deux), à chaîne ramifiée ;
(b) lorsque le nombre d'atomes de carbone est suffisant (à savoir un minimum de trois), cyclique (formant un groupe cycloalkyle en C₃-C_{q}, y compris les groupes cycloalkyle en C₃-C_{q} monocycliques ou bicycliques, groupes cycloalkyles qui peuvent être en outre pontés) ;
(c) lorsque le nombre d'atomes de carbone est suffisant (à savoir un minimum de quatre), partiellement cyclique ;
(d) saturé ou, lorsque le nombre d'atomes de carbone est suffisant (à savoir un minimum de deux), insaturé (formant un groupe cycloalcényle en C₃-C_{q}, un groupe cycloalcynyle en C₈, un groupe alcényle en C₂-C_{q}
ou un groupe alcynyle en C₂-C_{q}) ; et/ou
(e) former un composé « spiro », dans le cas où un substituant sur un groupe cycloalkyle est un autre composé cyclique fixé par un atome simple.

2. Composé selon la revendication 1, dans lequel :
A² et A³ représentent indépendamment une liaison simple, -0- ou -N(R⁸)- ;
Z¹ représente, dans chaque cas cité ci-dessus, =O, =NOR⁷, =NS(O)ₙN(R⁸)(R⁷), =NCN ou =C(H)NO₂ ;
A⁷ et A⁸ représentent indépendamment une liaison simple, -0- ou -N(R¹⁰)- ;
Z² représente, dans chaque cas cité ci-dessus, =O, =NOR⁹, =NS(O)ₙN(R¹⁰)(R⁹), =NCN ou =C(H)NO₂ ;
A¹² et A¹³ représentent indépendamment une liaison simple, -0- ou -N(R¹²)- ; et/ou
Z³ représente, dans chaque cas cité ci-dessus, =O, =NOR¹¹, =NS(O)ₙN(R¹²)(R¹¹), =NCN ou =C (H)NO₂.

3. Composé selon la revendication 1 ou la revendication 2, dans lequel n représente 2.

4. Composé selon l'une quelconque des revendications précédentes, dans lequel A représente G¹.

5. Composé selon l'une quelconque des revendications précédentes, dans lequel G¹ représente halogéno, cyano, -NO₂ ou -A¹-R⁷.

6. Composé selon la revendication 5, dans lequel G¹ représente -NO₂ ou -A¹-R⁷.

7. Composé selon l'une quelconque des revendications précédentes, dans lequel A¹ représente une liaison simple, -N(R⁸)A⁴- ou -OA⁵-.

8. Composé selon l'une quelconque des revendications précédentes, dans lequel A⁴ et A⁵ représentent indépendamment une liaison simple.

9. Composé selon l'une quelconque des revendications précédentes, dans lequel R⁷ et R⁸ sont choisis indépendamment parmi un hydrogène et un groupe alkyle en C₁-C₆, ce dernier groupe étant éventuellement substitué par un ou plusieurs substituants choisis parmi G³.

10. Composé selon l'une quelconque des revendications précédentes, dans lequel G³ représente un groupe halogéno.

11. Composé selon l'une quelconque des revendications précédentes, dans lequel R¹ représente un groupe phényle, naphtyle ou pyridyle éventuellement substitué.

12. Composé selon l'une quelconque des revendications précédentes, dans lequel X représente un groupe phényle ou pyridyle éventuellement substitué.

13. Composé selon l'une quelconque des revendications précédentes, dans lequel R³ et R⁴ représentent indépendamment G¹, un hydrogène ou un groupe phényle ou pyridyle éventuellement substitué.

14. Composé selon la revendication 13, dans lequel R³ et R⁴ représentent indépendamment un atome d'hydrogène ou un groupe phényle ou pyridyle éventuellement substitué.

15. Composé selon l'une quelconque des revendications précédentes, dans lequel l'un au moins parmi R³ et R⁴ représente un groupe phényle ou pyridyle éventuellement substitué, et éventuellement l'autre représente G¹ ou un hydrogène.

16. Composé selon l'une quelconque des revendications 13 à 15, dans lequel lorsque R³ ou R⁴ représente un groupe phényle ou pyridyle éventuellement substitué, alors les autres substituants sur le noyau benzène essentiel de l'indole de formule I tel que défini dans la revendication 1 (à savoir R², R⁵ et R³ ou R⁴ (le cas échéant)) représentent indépendamment G¹ ou un hydrogène.

17. Composé selon la revendication 16, dans lequel les autres substituants représentent un hydrogène.

18. Composé selon l'une quelconque des revendications 11 à 17, dans lequel les substituants facultatifs sont choisis parmi cyano, hétérocycloalkyle, halogéno, -NO₂, alkyle en C₁-C₆ (groupe alkyle qui peut être linéaire ou ramifié, cyclique ou partiellement cyclique, insaturé et/ou éventuellement substitué par un ou plusieurs groupes halogéno), -OR¹⁵ et -N(R¹⁵)R¹⁶, où R¹⁵ et R¹⁶ représentent indépendamment H ou un groupe alkyle en C₁-C₆ (groupe alkyle qui peut être éventuellement substitué par un ou plusieurs groupes halogéno).

19. Composé selon la revendication 18, dans lequel les substituants facultatifs sont choisis parmi halogéno, -NO₂, alkyle en C₁-C₆ (groupe alkyle qui peut être linéaire ou ramifié, cyclique ou partiellement cyclique, insaturé et/ou éventuellement substitué par un ou plusieurs groupes halogéno), -OR¹⁵ et -N(R¹⁵)R¹⁶, où R¹⁵ et R¹⁶ représentent indépendamment H ou un groupe alkyle en C₁-C₆ (groupe alkyle qui peut être éventuellement substitué par un ou plusieurs groupes halogéno).

20. Composé selon l'une quelconque des revendications précédentes, dans lequel R⁶ représente un atome d'hydrogène.

21. Composé selon l'une quelconque des revendications 1 à 20, ou un sel pharmaceutiquement acceptable de celui-ci, pour une utilisation comme produit pharmaceutique.

22. Formulation pharmaceutique comprenant un composé tel que défini dans l'une quelconque des revendications 1 à 20, ou un sel pharmaceutiquement acceptable de celui-ci, en mélange avec un adjuvant, un diluant ou un véhicule pharmaceutiquement acceptable.

23. Composé selon l'une quelconque des revendications 1 à 20, ou un sel pharmaceutiquement acceptable de celui-ci, pour le traitement d'une maladie dans laquelle l'inhibition de l'activité de la prostaglandine E synthase 1 microsomiale, la leukotriène C₄ synthase et/ou la protéine activant la 5-lipoxygénase est souhaitée et/ou nécessaire.

24. Composé selon la revendication 23, dans lequel l'inhibition de l'activité de la prostaglandine E synthase 1 microsomiale est souhaitée et/ou nécessaire.

25. Utilisation d'un composé tel que défini dans l'une quelconque des revendications 1 à 20, ou d'un sel pharmaceutiquement acceptable de celui-ci, pour la fabrication d'un médicament pour le traitement d'une maladie dans laquelle l'inhibition de l'activité de la prostaglandine E synthase 1 microsomiale, la leukotriène C₄ synthase et/ou la protéine activant la 5-lipoxygénase est souhaitée et/ou nécessaire.

26. Utilisation selon la revendication 25, dans laquelle la maladie est une inflammation.

27. Utilisation selon la revendication 26, dans laquelle la maladie est la maladie des intestins inflammatoires, le syndrome des intestins irritables, la migraine, le mal de tête, la douleur dans le bas du dos, la fibromyalgie, un trouble myofacial, une infection virale, une infection bactérienne, une infection fongique, la dysménorrhée, une brûlure, une opération chirurgicale ou dentaire, un cancer, l'athérosclérose, la goutte, l'arthrite, l'ostéoarthrite, l'arthrite juvénile, l'arthrite rhumatoïde, la fièvre, la spondylarthrite ankylosante, le lupus érythémateux aigu disséminé, la vasculite, la pancréatite, la néphrite, la bursite, la conjonctivite, l'iritis, la sclérite, l'uvéite, la cicatrisation d'une plaie, la dermatite, l'eczéma, le psoriasis, une crise, le diabète sucré, un trouble neurodégénératif, une maladie auto-immune, l'ostéoporose, l'asthme, la maladie pulmonaire obstructive chronique, la fibrose pulmonaire, un trouble allergique, la rhinite, un ulcère, une maladie cardiaque coronaire, la sarcoïdose, une douleur inflammatoire, le syndrome de l'hyperprostaglandine E, le syndrome de Bartter classique, la maladie de Hodgkin, le canal persistant, et toute autre maladie avec un composant inflammatoire, la maladie de Paget ou une maladie périodontale.

28. Produit de combinaison comprenant :
(A) un composé tel que défini dans l'une quelconque des revendications 1 à 20, ou un sel pharmaceutiquement acceptable de celui-ci ; et
(B) un autre agent thérapeutique qui est utile dans le traitement d'une inflammation,
dans lequel chacun des composés (A) et (B) est formulé en mélange avec un adjuvant, un diluant ou un véhicule pharmaceutiquement acceptable.

29. Produit de combinaison selon la revendication 28, qui comprend une formulation pharmaceutique comprenant un composé tel que défini dans l'une quelconque des revendications 1 à 20, ou un sel pharmaceutiquement acceptable de celui-ci, en mélange avec un adjuvant, un diluant ou un véhicule pharmaceutiquement acceptable.

30. Produit de combinaison selon la revendication 28, qui comprend un kit de parties comprenant les composants suivants :
(a) une formulation pharmaceutique comprenant un composé tel que défini dans l'une quelconque des revendications 1 à 20, ou un sel pharmaceutiquement acceptable de celui-ci, en mélange avec un adjuvant, un diluant ou un véhicule pharmaceutiquement acceptable ; et
(b) une formulation pharmaceutique comprenant un autre agent thérapeutique qui est utile dans le traitement d'une inflammation, en mélange avec un adjuvant, un diluant ou un véhicule pharmaceutiquement acceptable ;
composants (a) et (b) qui sont chacun fournis sous une forme qui convient à une administration en conjonction l'un avec l'autre.
